# EUROPEAN PATENT SPECIFICATION

(11) **EP 4 532 498 B1**
(45) Date of publication and mention of the grant of the patent: **29.10.2025**
(21) Application number: 23735588.8
(22) Date of filing: 29.05.2023
(51) Int. Cl.: C07D 473/16, C07D 473/34, A61K 31/52, A61P 3/00, A61P 25/28

(54) **HETEROCYCLIC COMPOUNDS FOR THE PREVENTION AND THERAPY OF CIRCADIAN RHYTHM DISORDERS**
HETEROCYCLISCHE VERBINDUNGEN ZUR PRÄVENTION UND THERAPIE VON STÖRUNGEN DES ZIRKADIANEN RHYTHMUS
COMPOSÉS HÉTÉROCYCLIQUES POUR PRÉVENIR ET TRAITER DES TROUBLES DU RYTHME CIRCADIEN

(30) Priority: 30.05.2022 CZ 20220227
(43) Date of publication of application: 09.04.2025
(73) Proprietor: Univerzita Palackého v Olomouci, 779 00 Olomouc (CZ); Fyziologicky ustav AV CR, v.v.i., 14200 Praha 4 (CZ)
(72) Inventor: VOLLER, Jiri, 63500 Brno (CZ); MIK, Vaclav, 78373 Grygov (CZ); DOKONALOVA, Marketa, 67976 Drnovice 105 (CZ); SLADEK, Martin, 14800 Praha 4 (CZ); SUMOVA, Alena, 15200 Praha 5 (CZ); HAJDUCH, Marian, 79305 Norbercany (CZ); STRNAD, Miroslav, 77900 Olomouc (CZ)
(74) Representative: Harber IP s.r.o.
(86) International application number: PCT/CZ2023/050029
(87) International publication number: WO 2023/232171

(56) References cited:
- WO-A1-2017/036434
- WO-A1-2018/196893
- WO-A1-2021/115502

## Description

### Field of Art

The invention relates to the use of heterocyclic compounds for the modulation of circadian rhythms of mammals, including humans, and their cells, tissues and organs. Such modulation is useful for the treatment and prevention of acute and chronic diseases of the circadian rhythm due to both genetic and socio-environmental factors.

### Background Art

The circadian clock (circadian rhythm) has evolved as an adaptation to a 24-hour solar day, and although its mechanism varies from organism to organism, it is one of the universal characteristics of life. The circadian clock allows to anticipate and prepare for regularly changing external conditions. However, it can also function in aperiodic conditions with its own genetically determined circadian period. The clock is entrainable by external cues (e.g. light, temperature, social interaction) and controls metabolic, physiological and behavioral rhythms. In mammals, circadian rhythms are controlled by a central pacemaker located in the suprachiasmatic nucleus of the hypothalamus (SCN), which is directly synchronized by light and regulates the local clock in other brain and peripheral tissues such as in the cerebral cortex, hippocampus, retina, liver, kidney, intestine, or pancreas. The main oscillator in the SCN, as well as the peripheral oscillators, are composed of interconnected transcriptional and (post) translational feedback loops (TTFLs) formed by families of clock genes such as *PER, BMAL1, CLOCK, REV-ERB, ROR a CRY.* The clock genes then rhythmically regulate a large group of predominantly tissue-specific clock-controlled genes with various functions, including regulation of metabolism, behavior, or cell division.

Interestingly, clock genes play a role in a number of other processes. For example, human BMAL1 contributes to the normal function of pancreatic beta cells and regulates glucose-stimulated insulin secretion. It further regulates the mTCRC1 signaling pathway by regulating *MTOR* and *DEPTOR* expression. Chemokine expression in Ly6C monocytes is also regulated by *ARNTL*/ *BMAL1* (Nguyen et al. 2013 [doi:10.1126/science.1240636]). It further regulates the expression of genes involved in hair growth (Watabe et al. 2013 [https://doi.org/10.1007/s00403-013-1403-0]). It also plays an important role in adult hippocampal neurogenesis by timing the entry of neuronal stem cells into the cell cycle. Human *PER2* plays a role in lipid metabolism (by suppressing the proadipogenic activity of PPARG) and in glucose metabolism (regulation of circulating insulin levels). *PER2* contributes to the maintenance of cardiovascular function by regulating the production of NO and vasodilating prostaglandins in the aorta. It also regulates the absorption of glutamate in synaptic vesicles, the absorption of fatty acids in the liver and is involved in the regulation of inflammatory processes. Clock-controlled genes and proteins can make up more than 20% of the total transcriptome and proteome, depending on the tissue.

Their rhythmic regulation allows cells, tissues and organs to perform physiological processes in a coordinated way, to anticipate and prepare for changes in the environment. Synchronization of individual oscillators in peripheral tissues by the SCN via hormonal and neuronal signals allows precise coordination and integration of various physiological functions.

Dysfunctions of the circadian rhythm in terms of changes in period, phase or amplitude at the level of cells, tissues, organs or the whole body as well as phase mismatch between oscillators in individual tissues/organs or between biological and external time lead to disruption of homeostasis, resulting in various pathologies.

Examples of conditions associated with circadian rhythm dysfunction or impairment are: depressive disorder, unipolar depression, bipolar disorder, seasonal affective disorder, dysthymia, anxiety disorder, schizophrenia, Alzheimer's disease, REM sleep disorders, FASPS, ASPD, delayed sleep syndrome phases and free-running sleep phases, hypersomnia, parasomnia, narcolepsy, nocturnal enuresis, metabolic syndrome and obesity, hyperinsulinemia, type 2 diabetes and restless legs syndrome (Jones et al. 2013 [doi: 10.1016 / j.expneurol.2012.07.012); Marcheva et al., 2010 [doi: 10.1038 / nature09253]; Kalsbeek, 2013 [doi: 10.2337 / db12-0507]; Suli et al., 2019 [doi: 10.1016 / j.trecan.2019.07.002]). Growing evidence also points to an association of circadian rhythm disorders and cancer (Ballesta et al. 2017 [doi: 10.1124 / pr.116.013441]; Panda 2019 [doi: 10.1038 / s41574-018-0142-x]).

Circadian rhythm disorders and diseases (Fishbein et al. 2021 [doi:10.1172/JCI148286], Zee et al. 2013 [doi:10.1212/01.CON.0000427209.21177.aa]) include those in which the primary cause is dysfunction or misalignment of the circadian clock (hereditary advanced sleep phase syndrome - FASPS, advanced sleep phase disorder - ASPD, irregular sleep phase syndrome and free-running sleep phase syndrome, jet lag disorder, shift-work disorder, social jet lag), as well as those with a circadian rhythm dysfunction caused by a disease and contributing to the pathophysiology of the disease. Mild cognitive impairment, Alzheimer's disease and Smith-Magenis syndrome are examples of diseases where the effect on the circadian rhythm is variable - phase advance and the corresponding abnormal sleep-wake cycle is present only in some patients (Naismith et al. 2014 [doi: 10.3233/JAD -131217], Liguori et al. 2014 [doi:10.1001/jamaneurol.2014.2510], Nováková et al. 2012 [10.1210/jc.2011-2750]).

In the absence of external stimuli (for example in constant darkness), the endogenous period of circadian rhythm in humans is on average slightly longer than 24 hours. In completely blind subjects unable to synchronize with light, such a period often persists chronically. As a result, their sleep-wake cycle is free-running with respect to external time, which is often associated with negative metabolic, cognitive and emotional consequences.

The discrepancy of the circadian clock with the external time is also a typical feature of modern life. Air travel allows fast movement across time zones, which results in jet lag. Artificial light allows activity independently of natural light. Natural light effectively synchronizes the central oscillator in the SCN. Personal preference for a specific sleep phase (chronotype) and social factors influence the timing of the individual activity and these factors are often in conflict, resulting in a so-called social jet lag (i.e. a chronic difference between the sleep phase on free days and on working days), which has a negative effect on health (Roenneberg et al, 2007 [doi: 10.1016/j.smrv.2007.07.005]). A particularly important factor causing circadian desynchronization is shift work due to its prevalence (more than 17% of the EU workforce is night workers). The result is frequent sleep problems, fatigue and reduced manual and mental performance. Jet lag disorder, social jet lag and shift work disorder significantly affect physiological functions and increase the incidence of lifestyle diseases (Roenneberg et al., 2012 [doi: 10.1016/j.cub.2012.03.038]; Roenneberg and Merrow, 2016 [doi: 10.1016/j.cub.2016.04 .011]). Jet lag disorder and disorder associated with shift work are internationally classified as sleep disorders (G47.25 (circadian rhythm sleep disorder, jet-lag type) and G47.26 (circadian rhythm disorder, shift-work type), Thorpy, 2012 [doi: 10.1007/s13311-012-0145-6]). Frequent shift workers also have an increased risk of depression, metabolic syndrome (Biggi et al., 2016 [doi: 10.1080/07420520802114193]), breast, prostate and rectal cancer (Sulli et al., 2019 [doi: 10.1016/j.trecan.2019.07.002]). Symptoms of jet lag and social jet lag include not only sleep disturbances (interrupted sleep, problems with (re)falling asleep and staying asleep, waking up at inappropriate times) and fatigue during the day, but also mood changes, cognitive problems (confusion, lack of concentration, reduced mental agility), nausea, dizziness, anxiety, headaches, digestive problems, changes in stool frequency and consistency, and decreased interest in surroundings and food. These problems cannot be effectively cured using hypnotics. On the other hand, compounds capable of inducing or speeding up the synchronization of internal and external time by acting directly on the cellular components of the circadian oscillator are a suitable means.

Current approaches to the treatment of circadian rhythm disorders and diseases are dominated by various variants of bright light therapy and the use of melatonin or synthetic melatonin receptor agonists. However, these methods also have significant disadvantages. Bright light therapy is considered very inconvenient for many users because it requires exposure to very high levels of light in precise time windows every day (Zee et al., 2013 [doi: 10.1212/01.CON.0000427209.21177.aa.]). It cannot be used in blind patients. Melatonin is only effective in subset of patients with sleep disorders. It doesn't have a significant effect, for example, on patients suffering from FASPS, ASPD or jet lag after traveling westwards. It may cause unwanted drowsiness. In addition, there is great individual variability in response to melatonin. Another disadvantage of light or melatonin is that their effect on the rhythm phase is directly dependent on the instantaneous phase of the central clock in the SCN. For example, light applied during a subjective day has no effect on the SCN phase, as it is in that moment in non-responsive zone of the phase-response curve, so it is necessary to time the therapy to the early morning or late evening hours.

Therefore, a direct effect on the molecular circadian mechanism could be a new effective way to adjust circadian rhythms. Such therapy would be beneficial in a number of disorders associated with various types of circadian dysfunctions requiring proper synchronization, period length adjustment, a one-time new phase adjustment, or increased overall rhythm integrity. In addition, unlike light or melatonin therapies, therapy using agents that directly target the molecular mechanism does not require SCN, would be equally effective day and night, and its timing in general is unlikely to significantly affect its effect, which can be easily modulated by dose adjustment.

### Disclosure of the Invention

The invention relates to heterocyclic compounds of general formula I 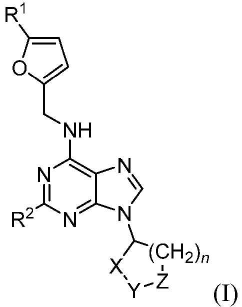 and their pharmaceutically acceptable salts,
wherein
each of X, Y, Z is selected from CH₂ and O, wherein at most one of X, Y, Z is O;
*n* is 1 or 2 or 3;
R¹ is H or C1-C4 alkyl;
R² is selected from H, Cl, C1-C4 alkoxy and NR²¹R²²,
wherein R²¹ and R²² are independently selected from H, C3-C6 cycloalkyl and C1-C8 alkyl, wherein one carbon atom in the C1-C8 alkyl may optionally be replaced by one nitrogen atom or one oxygen atom;
or wherein R²¹ is H or C1-C4 alkyl and R²² is selected from C6-C10 aryl-methyl, C6-10 aryl-ethyl, C3-C6 heteroaryl-methyl, C3-C6 heteroaryl-ethyl, wherein the heteroaryl group contains 1-2 heteroatoms selected from O, S, N;
or wherein R²¹ and R²² together with the nitrogen atom to which they are bound form a 3- to 8-membered ring optionally containing one further heteroatom selected from O, S, N;
for use for modulating circadian rhythms of mammals, especially humans.

When the compound of formula I contains chiral centre(s), the present invention also includes optically active isomers, mixtures thereof and racemates.

The compounds of formula I are for useful for the prevention and treatment of circadian rhythm dysfunctions, disorders and diseases, both acute and chronic, due to their ability to modulate circadian rhythm.

The present invention also relates to compounds of formula Ia 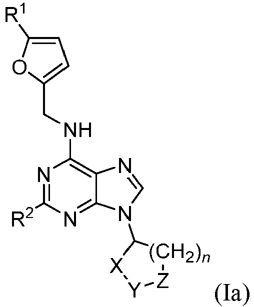 and their pharmaceutically acceptable salts,
wherein
- *n* is 1, X is CH₂ and each of Y, Z is selected from CH₂ and O, wherein exactly one of Y and Z is O; or
- *n* is 2, X is CH₂ and each of Y, Z is selected from CH₂ and O, wherein at most one of Y and Z is O; or
- *n* is 3, X is CH₂ and each of Y, Z is selected from CH₂ and O, wherein at most one of Y and Z is O;
   and

R¹ is H or C1-C4 alkyl;
R² is selected from H, Cl, C1-C4 alkoxy and NR²¹R²²;
wherein R²¹ and R²² are independently selected from H, C3-C6 cycloalkyl and C1-C8 alkyl, wherein one carbon atom in the C1-C8 alkyl may optionally be replaced by one nitrogen atom or one oxygen atom;
or wherein R²¹ is H or C1-C4 alkyl and R²² is selected from C6-C10 aryl-methyl, C6-10 aryl-ethyl, C3-C6 heteroaryl-methyl, C3-C6 heteroaryl-ethyl, wherein the heteroaryl group contains 1-2 heteroatoms selected from O, S, N;
or wherein R²¹ and R²² together with the nitrogen atom to which they are bound form a 3- to 8-membered ring optionally containing one further heteroatom selected from O, S, N.

Preferably, when *n* is 2, X or Z is O.

Preferably, when *n* is 3, X is O.

In some embodiments, when *n* is 1, all of X, Y, Z are CH₂.

In some embodiments, when *n* is 1, X or Y is O.

Preferably, the *N*9 substituent (the cycle containing -CH-X-Y-Z-(CH₂)ₙ-) is selected from the group comprising cyclopentyl, tetrahydrofuran-2-yl, tetrahydrofuran-3-yl, tetrahydro-2*H-*pyran-2-yl, tetrahydro-2*H*-pyran-3-yl, tetrahydro-2*H*-pyran-4-yl, oxepan-2-yl.

Preferably, R¹ is H or CH₃.

In some embodiments, R²¹ and R²² are independently selected from C1-C4 alkyls.

In some embodiments, R²¹ is H and R²² is C1-C4 alkyl.

In some embodiments, R²¹ is H and R²² is C1-C8 alkyl, wherein one carbon atom is replaced by one nitrogen atom or one oxygen atom. Preferably, the carbon atom which is replaced by the oxygen atom or nitrogen atom is a terminal carbon atom.

In some embodiments, R²¹ is H and R²² is furfuryl, benzyl or cyclopentyl.

In some embodiments, R²¹ and R²² together with the nitrogen atom to which they are bound form morpholinyl, thiomorpholin-4-yl or pyrrolidin-1-yl.

Preferably, R² is selected from chloro, isopropoxy, methylamino, ethylamino, propylamino, cyclopentylamino, benzylamino, furfurylamino, 2-aminoethylamino, 6-aminohexylamino, 2-(methylamino)ethylamino, 2-(dimethylamino)ethylamino, 2-hydroxyethylamino, 3-methoxypropylamino, dimethylamino, diethylamino, dipropylamino, 2-aminoethyl(methyl)amino, pyrrolidin-1-yl, morpholinyl, thiomorpholin-4-yl.

Alkyl group can be a linear or branched alkyl. C1-C4 alkyl may be selected from methyl, ethyl, propyl, isopropyl, butyl, isobutyl, *tert*-butyl.

Alkoxy group may be selected from methoxy, ethoxy, propoxy, isopropoxy, butoxy, isobutoxy, tert-butoxy.

Modulation of circadian rhythms by compounds of the general formula I includes in particular the increasing (extending) of the period of the circadian rhythm and the associated shift of the phase of the circadian rhythm.

The term "rhythm modulation" (or circadian rhythm modulation, or modulation of circadian rhythms) herein means increasing (extending) the period of a cell's, tissue's or organism's circadian rhythm and/or shifting the phase of a cell's, tissue's or organism's circadian rhythm.

"Increasing of the period of the circadian rhythm" means the increasing (extending) of the internal period of the circadian rhythm of cells, tissues or an organism compared to the original period of the circadian rhythm. In the case of diseases with a shortened period of the circadian rhythm, the increasiong of the period serves to normalize the period to approximately 24 hours.

"Phase shift of circadian rhythm" means the advance or delay of the circadian rhythm of cells, tissues or organisms compared to their original circadian phase. The phase shift of the circadian rhythm is achieved by temporarily increasing its period. The goal of circadian rhythm phase shifting is to adjust the phase of the internal circadian rhythm so that it aligns better with the desired phase of this rhythm. The required phase may coincide with the phase of solar time or the phase of the internal circadian rhythm suitable for performing a therapeutic intervention.

The invention is based on the finding that heterocyclic compounds of formula I modulate the activity and concentration of molecular components of the mammalian cellular circadian oscillator, and increase the period of the circadian rhythm. By temporarily increasing the period, it is also possible to achieve a phase shift of the circadian rhythm. Heterocyclic compounds of formula I strongly influence the circadian oscillator at concentrations at which corresponding compounds without a substitution at position 9 of the purine heterocycle are inactive. This finding leads to a number of therapeutic applications.

An increase of the period of the circadian rhythm, and in the case of single or short-term administration (< 5 days) also a shift of the phase of the circadian rhythm, achievable by the administration of compounds of formula I is able to treat diseases of circadian rhythms.

Increasing the period of the circadian cycle using the compound of formula I has a therapeutic effect especially for diseases where the period of the circadian cycle is shortened and/or the phase of the circadian rhythm is advanced. Such diseases are mainly hereditary advanced sleep phase syndrome - FASPS, advanced sleep phase disorder - ASPD, irregular sleep phase syndrome and free running sleep disorder. Long-term administration (more than 5 days) is advantageous here.

Increasing the period of the circadian cycle with the compound of formula I has a therapeutic effect especiallyfor diseases where the shortening of the period of the circadian cycle and/or advancing of the phase of the circadian rhythm is caused by the disease and further contributes to the progression of the disease and/or to the deterioration of the quality of life. Such diseases are mainly abnormal circadian activity associated with neurodegeneration such as mild cognitive impairment and Alzheimer's disease, or developmental disorders such as Smith-Magenis syndrome. Here, long-term therapeutic administration (more than 5 days) is advantageous to persons who exhibit abnormal circadian activity (abnormal sleep-wake cycle) as a result of a shortened period of the circadian cycle and/or advanced phase of the circadian rhythm.

The shift of the phase of the circadian rhythm due to the short-term increase of the period after a single or short-term administration of the compound of formula I allows prevention or treatment of diseases and conditions caused by the mismatch of the phase of the person's own circadian rhythm with the external environment. For prophylactic or therapeutic effects against jet lag disorder, social jet lag, and/or shift work disorder, it is advantageous to administer the compound of formula I on a single or short-term basis (single or repeated for 1 to 5 days). The advantage of a one-time and short-term administration is a smaller burden on the body, including the metabolic systems, with the drug.

Jet lag disorder is classified as a disease: circadian rhythm sleep disorder, jet-lag type. Shift work disorder is classified as a disease: circadian rhythm disorder, shift-work type. Social jetlag is defined as the discrepancy between biological time, determined by our internal body clock, and social times, mainly dictated by social obligations such as school or work. Social jet lag has a high prevalence in developed countries and represents a substantial health risk. It leads to obesity, diabetes and cardiovascular morbidity (Caliandro R. et al.: 10.3390/nu13124543).

Modulation of circadian rhythms by compounds of formula I has major advantages over the use of hypnotics, which are used with partial and variable success in the therapy of sleep disorders due to disruption of circadian rhythms. The compounds of formula I also treat insomnia symptoms and symptoms of disturbed rhythm. They do not directly affect alertness and attention. Unlike hypnotics, these compounds can also be administered preventatively. For example, synchronizing the internal clock with the objective time at the destination in order to avoid jet lag may be partially or fully performed before the trip begins. Similarly, it is possible to use preventative administration to speed up the alignment of the circadian rhythm during a planned change of activity (e.g. preparation for shift work or other social activities). An advantage over melatonin, for example, is the ability to act on the components of the circadian oscillator in cells and tissues outside the SCN, thus accelerating the normalization of the circadian rhythm.

Phase shift induced by single or short-term (up to 5 days) administration of a compound of formula I can also be used to synchronize the phase of the circadian rhythm, i.e. the optimal circadian time of application of a veterinary or human medicine treatment in terms of its efficacy and safety, with the solar time suitable for the application of therapy from the point of view of the organization of the working time of persons applying the therapy. Such treatment can be, for example, surgery or chemotherapy.

The compounds can be administered prophylactically or therapeutically as such or in the form of pharmaceutical preparations, in an amount that is effective against the said diseases, whereby in a patient in need of such treatment, the compound is used preferably in the form of a pharmaceutical preparation. The applied daily dose is preferably between 1 and 100 mg/kg.

The present invention further provides a pharmaceutical composition comprising at least one compound of formula I and at least one pharmaceutically acceptable carrier. The compound of formula I can occur in compositions, inter alia, in the form of pharmaceutically acceptable salts or solvates.

In a preferred embodiment, these compositions are dosage forms for oral administration. In another important embodiment, the compositions are dosage forms for transdermal, inhalation or nasal application. In other embodiments, the compositions are dosage forms for other forms of parenteral administration, such as intravenous, intramuscular or subcutaneous administration.

Pharmaceutically acceptable salts of compounds of formula I are formed by protonation of one of the nitrogen atoms with an inorganic or organic acid. Examples of salts with inorganic acids include hydrochlorides, hydrobromides, hydroiodides, sulfates, nitrates, phosphates, hydrogen phosphates, dihydrogen phosphates, carbonates, hydrogen carbonates, perchlorates. Examples of salts with organic acids include salts with linear or branched acids of 2 to 20 carbons, such as lactate, oxalate, fumarate, tartrate, malate, maleate, citrate, succinate, glycolate, nicotinate, benzoate, salicylate, ascorbate, pamoate; sulfonate, methanesulfonate, ethanesulfonate, 2-hydroxyethanesulfonate, benzenesulfonate, *p*-toluenesulfonate, 2-naphthalenesulfonate, 3-phenylsulfonate and camphorsulfonate, aspartate or glutamate.

Pharmaceutical compositions:
Dosage forms for oral administration include coated and uncoated tablets, soft and hard gelatin capsules, matrix tablets, solutions, emulsions, suspensions, syrups, powders and granules for reconstitution, chewable and effervescent tablets. The therapeutic preparation for oral administration contains from 1 to 95 wt.% of the active substance - compound of formula I, preferably 1-20 wt.% of the compound.

Dosage forms for peroral administration include tablets, coated tablets, capsules, solutions and syrups. Dosage forms for parenteral administration include solutions, emulsions, suspensions and dispersions, powders and granules for reconstitution. Other dosage forms contemplated include suppositories, forms for transdermal penetration, implants, and forms for insufflation and inhalation.

Pharmaceutical compositions according to the present invention are prepared in a known manner, e.g. by conventional mixing, granulation, coating, dissolving or lyophilization processes.

Solutions of active compounds are preferably used, as well as suspensions or dispersions, especially isotonic aqueous solutions, suspensions or dispersions, which can be prepared before use, e.g. in the case of lyophilized preparations containing the active compound alone or with a carrier such as mannitol. Pharmaceutical compositions may be sterilized and/or contain excipients such as preservatives, stabilizers, wetting agents and/or emulsifiers, solubilizing agents, salts for regulating osmotic pressure and/or buffers. They are prepared in a known manner, e.g. by ordinary dissolution or lyophilization. Said solutions or suspensions may contain viscosity-increasing substances, such as sodium carboxymethylcellulose, dextran, polyvinylpyrrolidone or gelatin.

Oil suspensions contain as an oil component vegetable, synthetic or semi-synthetic oils usual for injection purposes. The oils which may be used are especially liquid fatty acid esters where the fatty acid has 8-22, preferably 12-22 carbon atoms, e.g. lauric acid, tridecanoic acid, myristic acid, pentadecanoic acid, palmitic, margaric, stearic, arachidonic and behenic acids, or is a corresponding unsaturated acid, e.g. oleic, elaidic, euric, brasidic and linoleic acids, possibly with the addition of antioxidants, e.g. vitamin E, beta-carotene or 3,5-di-*tert-*butyl-4-hydroxytoluene. The alcohol component of these fatty acid esters has no more than 6 carbon atoms and is mono- or polyhydric, e.g. mono-, di- or trihydric alcohols such as methanol, ethanol, propanol, butanol or pentanol and their isomers, but mainly glycol and glycerol. Fatty acid esters are preferably e.g. ethyl oleate, isopropyl myristate, isopropyl palmitate, "Labrafil M 2375" (polyoxyethylene glycerol trioleate, Gattefoseé, Paris), "Labrafil M 1944 CS" (unsaturated polyglycol glycerides prepared by alcoholysis of apricot oils cores and composed of glycerides and polyethylene glycol esters; Gattefoseé, Paris), "Labrasol" (saturated polyglycol glycerides prepared by alcoholysis of TCM and composed of glycerides and polyethylene glycol esters; Gattefoseé, Paris) and/or "Miglyol 812" (triglyceride of saturated fatty acids with a chain length of C8 to C12 from Hűls AG, Germany) and especially vegetable oils such as cottonseed oil, almond oil, olive oil, castor oil, sesame oil, soybean oil and especially groundnut oil.

Preparation of the injectables is carried out under sterile conditions in the usual way, e.g. by filling into ampoules or vials and sealing the packages.

E.g. pharmaceutical compositions for oral use can be obtained by mixing the active compound with one or more solid carriers, possibly granulating the resulting mixture and, if desired, processing the mixture or granules into tablets or coated tablets by adding other neutral substances.

Suitable carriers are especially fillers such as sugars, e.g. lactose, sucrose, mannitol or sorbitol, cellulose preparations and/or calcium phosphates, preferably calcium phosphate or calcium hydrogen phosphate, and binders such as starches, preferably corn, wheat, rice or potato starch, methyl cellulose, hydroxypropyl methyl cellulose, sodium salt of carboxymethyl cellulose and/or polyvinylpyrrolidine, and/or if desired, disintegrators such as the above-mentioned starches and also carboxymethyl starch, cross-linked polyvinylpyrrolidine, alginic acid and its salts, preferably sodium alginate. Other neutral substances are flow regulators and lubricants, preferably salicylic acid, talc, stearic acid and its salts such as magnesium and/or calcium stearate, polyethylene glycol or its derivatives. The cores of the coated tablets may be coated with suitable coatings which may be resistant to gastric juice. The suitable coatings include concentrated solutions of sugars, which may contain gum arabic, talc, polyvinylpyrrolidone, polyethylene glycol and/or titanium dioxide. Further, coatings can be coating solutions in suitable organic solvents or mixtures of solvents for the preparation of coatings resistant to gastric juice, solutions of suitable cellulose preparations such as acetyl cellulose phthalate or hydroxypropyl methyl cellulose phthalate. Dyes or pigments are mixed into tablets or coated tablets, for example to identify or characterize different doses of the active ingredient.

Pharmaceutical compositions for oral administration are also hard capsules made of gelatin or soft closed capsules made of gelatin and plasticizers such as glycerol or sorbitol. Hard capsules may contain the active compounds in the form of granules, mixed for example with fillers such as corn starch, binders or lubricants such as talc or magnesium stearate, and with stabilizers. In soft capsules, the active compound is preferably dissolved or suspended in suitable liquid substances of a neutral nature, such as lubricating grease, paraffin oil or liquid polyethylene glycol or esters of fatty acids and ethylene or propylene glycol, while it is also possible to add stabilizers and detergents such as polyethylene sorbitan fatty acid esters .

Other forms for oral use are, for example, syrups prepared in a conventional manner, which contain the active compound, for example, in a suspended form and in a concentration of about 1 to 20%, preferably about 10% or a similar concentration that allows administration of a suitable individual dose, for example when measured 5 or 10 ml. Other forms are e.g. powder or liquid concentrates for preparing cocktails, e.g. in milk. Such concentrates may also be packaged in unit dose quantities.

Pharmaceutical compositions that can be used rectally are, for example, suppositories that contain a combination of an active compound and a base. Suitable bases are, for example, natural or synthetic triglycerides, paraffin hydrocarbons, polyethylene glycols or higher alcohols.

Compositions suitable for parenteral administration are aqueous solutions of the active compound in a water-soluble form, e.g. a water-soluble salt or an aqueous injectable suspension that contains viscosity-increasing substances, e.g. sodium carboxymethyl cellulose, sorbitol and/or dextran, and stabilizers where that is appropriate. The active compound may also be present in the form of a lyophilisate together with excipients where appropriate and may be dissolved prior to parenteral administration by adding suitable solvents. Solutions that are used for parenteral application can also be used, for example, for infusion solutions. Preferred preservatives are antioxidants such as ascorbic acid or microbicides such as sorbic or benzoic acid.

Tinctures and solutions usually contain a water-ethanol base to which humectants are mixed to reduce evaporation, such as polyalcohols, e.g. glycerol, glycols and/or polyethylene glycol, as well as lubricants such as esters of fatty acids and lower polyethylene glycols, i.e. lipophilic substances soluble in water mixtures replacing fatty substances removed from the skin with ethanol and, if necessary, also other excipients and additives.

### Brief description of drawings

Figure 1. Comparison of the effect of compound 4 and *N*⁶-furfuryladenine on the period of the circadian rhythm in the U2OS line transduced with the Bmal1-Luc reporter (concentration curve, mean±SD of 8 samples/concentration).
Figure 2. Demonstration of the effect of compound 4 on the phase of the circadian rhythm in the U2OS line transduced with the Bmal1-Luc reporter.

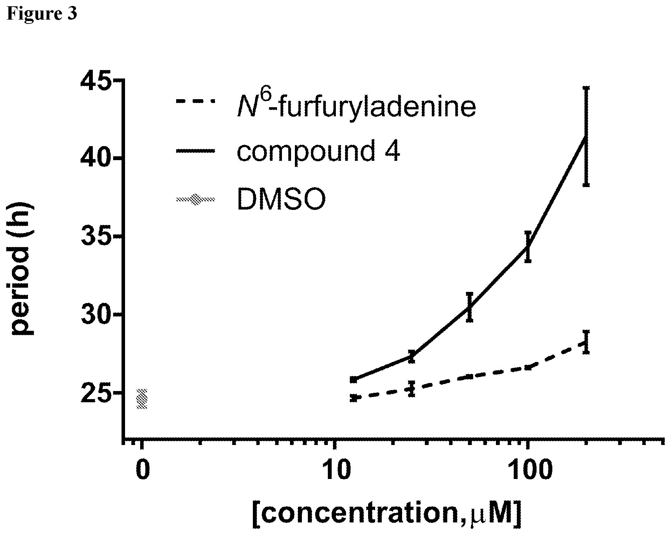

Figure 3. Comparison of the effect of compound 4 and *N*⁶-furfuryladenine on the period of the circadian rhythm in the NIH3T3 line transfected with the Per2-Luc reporter (mean of 4 samples ± SD).
Figure 4. Compound 4 increases the circadian rhythm period in organotypic explants of hypothalamic suprachiasmatic nucleus, the central oscilator. The recordings show luminescence directly proportional to the PER2 protein levels. (Example 14)

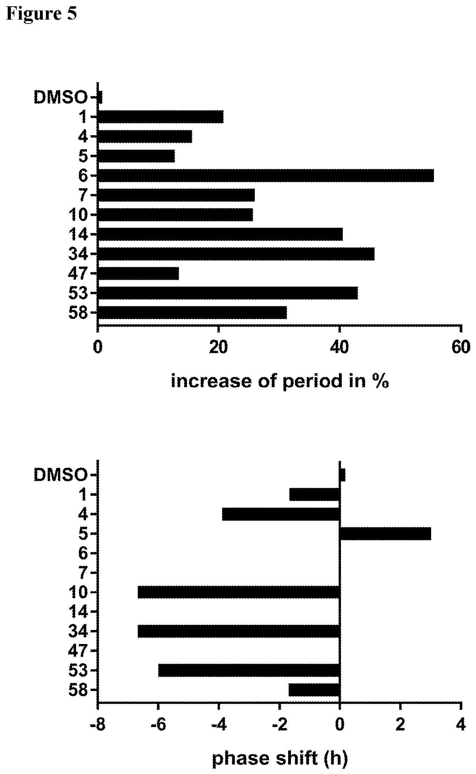

Figure 5. Compounds of formula I modulate the parameters of the circadian clock in the suprachiasmatic nucleus, the central circadian oscillator. It increases the period (left; measured as the ratio of period during 72 h after and 72 h before application of a compound) and induces phase shift (right; quantified by fitting a cosine curve before the application of a compound, extrapolating it beyond the time of application and subtracting the actual and extrapolated phases of the first full circadian cycle corrected by endogenous period of the explant, while designating phase advance as positive and phase delay as negative number in hours). (Example 14)

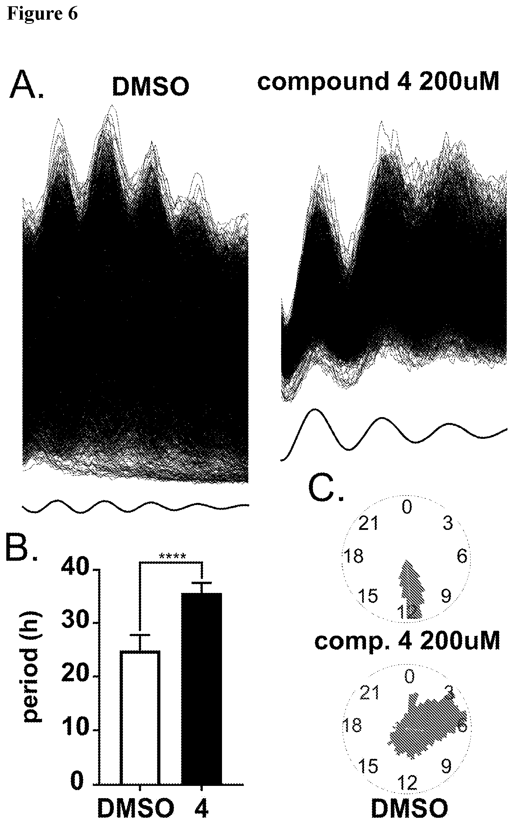

Figure 6: Compound 4 affects the circadian clock in the explanted choroid plexus (CHP). (A) Individual PER2::Luc traces in analysed single-cell-sized regions of interest (ROIs) of explanted choroid plexus (CP) cultivated over 5 days ex vivo in the presence of DMSO (left) or compound 4 (right). Cosine curve below the traces indicates average oscillation across the whole explant; see dramatic increase of period in the presence of 200uM compound 4. (B) Mean ± SD of period of PER2::Luc after treatment with 200uM compound 4 or DMSO (vehicle) is significantly (t-test, P < 0.0001) increased in CP explant treated with compound 4. (C) Phase (expressed as polar histogram of analysed ROIs, with white arrow indicating resulting Rayleigh vector) is dramatically affected by presence of compound 4 (upper) in comparison with DMSO (lower). (Example 15)

### Examples

The invention is further illustrated using the following examples, which should not be construed as limiting. Unless otherwise stated, all percentages and similar quantities are by weight.

**List of abbreviations and acronyms :**

| *Abbreviation* | *Meaning* |
|---|---|
| app. | Approximately |
| ASPD | Advanced Sleep Phase Disorder |
| ASPS | Advanced Sleep Phase Syndrome |
| bd | Broad doublet |
| bs | Broad singlet |
| CHCl₃ | Chloroform |
| ChP | Choroid plexus |
| cP | Cyclopentyl |
| d | Doublet |
| dd | Doublet of doublets |
| ddd | Doublet of doublets of doublets |
| DHF | 2,3-Dihydropyran |
| DHP | 3,4-Dihydro-2H-pyran |
| DIAD | Diisopropyl azodicarboxylate |
| DIPEA | *N,N-*diisopropylethylamine |
| DMSO | Dimethyl sulfoxide |
| DMSO-*d*₆ | Deuterated dimethyl sulfoxide |
| Eq. | Equivalent |
| ESI⁺ | Electrospray ionization in positive mode |
| Et₂O | Diethylether |
| EtOAc | Ethyl acetate |
| EtOH | Ethanol |
| FASPS | Familial Advanced Sleep Phase Disorder |
| fur | Furan |
| H₂SO₄ | Sulfuric acid |
| HPLC | High-performance liquid chromatography |
| HRMS | High resolution mass chromatography |
| iPrOH | Propan-2-ol, isopropanol |
| LC | Liquid chromatography |
| m | Multiplet |
| MeOH | Methanol |
| MS | Mass chromatography |
| MW | Microwave |
| NaH | Sodium hydride |
| Na₂SO₄ | Anhydrous sodium sulfate |
| n-PrOH | Propan-1-ol |
| NMR | Nuclear magnetic resonance |
| PDA | Diode-array detector |
| PE | Petroleum ether |
| pent | Pentet |
| PPh₃ | Triphenylphosphine |
| ppm | Parts per million |
| pur | Purine |
| q | Quartet |
| qd | Quartet of doublets |
| rt | Room temperature |
| s | Singlet |
| sept | Septet |
| t | Triplet |
| td | Triplet of doublets |
| tt | Triplet of triplets |
| TEA | Triethylamine |
| TFA | Trifluoroacetic acid |
| TOF | Time-of-flight |
| THF | Tetrahydrofuran |
| THO | 2,3,4,5-Tetrahydrooxepine |
| THP | Tetrahydro-2*H*-pyran |
| UV | Ultraviolet |
| RP | Revers phase |
| SCN | Suprachiasmatic nucleus |

### Materials and methods:

Starting material was obtained from commercial sources (Acros Organics, Sigma Aldrich, VWR, etc.) or were prepared as described below. Thin-layer chromatography was carried out on silica 60 Å plates with F₂₅₄ fluorescent indicator (VWR) using CHCl₃/MeOH (9:1, v/v,) or PE/EtOAc (1:1, v/v) as a developing system and the spots were detected by UV light (254 and 365 nm) and/or 6% vanillin in absolute EtOH containing 1 % (v/v) of H₂SO₄. Column chromatography purifications were carried out by irregular shaped silica 40-63 µm (VWR Chemicals) with gradient elution using CHCl₃/MeOH or PE/EtOAc as a mobile phase or isocratically with Et₂O. Preparative HPLC was performed on 1290 Infinity II Autoscale preparative LC/MSD System (Agilent) with variable wavelength UV detector 1260 Infinity II and single quadrupole mass spectrometer with electrospray ionization source LC/MSD. Compounds were purified on Agilent 5 Prep-C18 column (50 × 21.2 mm, particle size 5 µm or 100 × 21.2 mm, particle size 5 µm) using water or 0.1% acetic acid as solvent A and MeOH or acetonitrile as organic modifier (solvent B) with a flow rate 20 ml/min. The chromatographic purity and molecular mass of prepared compounds was determined using an ACQUITY UPLC^{®} H-Class System (Waters) linked simultaneously to photodiode array detector ACQUITY UPLC PDA (Waters) and benchtop single quadrupole mass spectrometer QDa (Waters). Samples were dissolved in DMSO to a concentration 1 mg/100 µl and diluted 1000× into methanol. Samples (10 µl) were injected on a RP-column Symmetry C18 (150 mm × 2.1 mm × 3.5 µm, particle size 5 µm, Waters) and separated at a flow rate of 0.2 ml/min with the following binary gradient: 0 min, 10% B; 0-24 min, a linear gradient to 90% B, 10 min, followed by 10 min isocratic elution of 90% B. At the end of the gradient, the column was re-equilibrated to initial conditions. 15 mM formic acid adjusted to pH 4.0 by ammonium hydroxide was used as solvent (A) and methanol as the organic modifier (solvent B). The eluent was introduced into the PDA detector (scanning range 190-500 nm, with 1.2 nm resolution) and an ESI source (source temperature 110 °C, capillary voltage +3.0 kV, cone voltage +20 V, desolvation temperature 250 °C). Nitrogen was used both as desolvation gas (500 l/h) as well as cone gas (50 l/h). The data was obtained in positive (ESI+) ionization mode in the 50-1000 *m*/*z* range. High resolution mass spectra were obtained on 1290 Infinity II Autoscale preparative LC/MSD System (Agilent) coupled simultaneously to 6230 time-of-flight LC/MS mass spectrometer (LC/TOF, Agilent) with electrospray ionization source Dual AJS ESI under following conditions: gas temperature: 260 °C, drying gas: 8 l/min, nebulizer: 35 psi, sheath gas temperature: 350 °C, sheath gas flow: 11 l/min, capillary voltage: 3500 V, nozzle voltage: 300 V. The masses were measured in Full scan mode from 100-1700 *m*/*z* and analysed by MassHunter software. NMR spectra were recorded on ECA-500 spectrometer (Jeol) operating at a frequency of 500 MHz (¹H) and 125 MHz (¹³C) or ECZ/R 400 spectrometer (Jeol) operating at a frequency of 400 MHz (¹H) and 100 MHz (¹³C), 375 MHz (¹⁹F), respectively. Samples were prepared by dissolving compounds in DMSO-*d*₆ and chemical shifts were calibrated to residual solvent peak (DMSO, 2.49 ppm for ¹H) and DMSO-*d*₆ (39.5 ppm for ¹³C), respectively. Chemical shifts in ¹⁹fluorine spectra were referenced to CCl₃F (0 ppm).

### Synthesis of compounds of formula I and/or Ia:

Procedure for the preparation of the compounds is summarized in Scheme 1 below, and further described below.

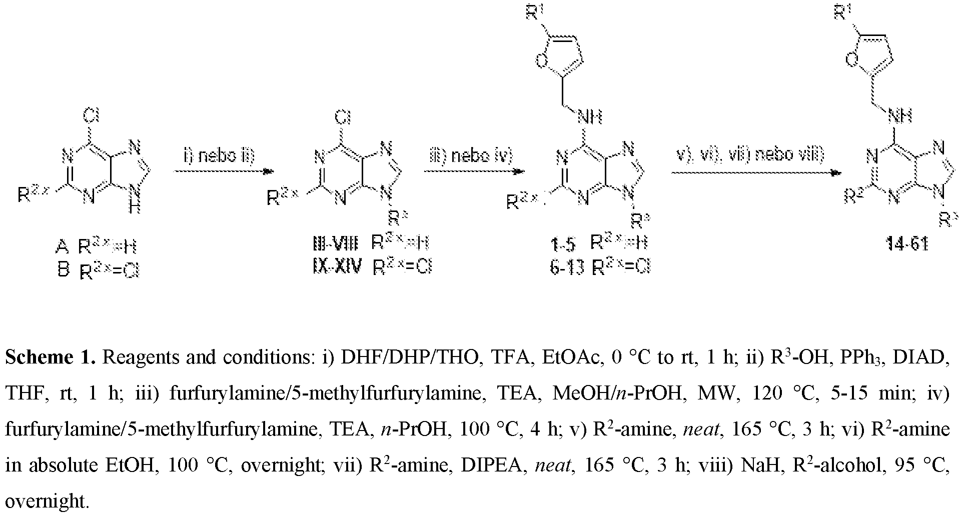

**Table 1. Intermediates (III-XIV).**

| **Compound number** | **PURINE SUBSTITUENT** | | | **MS ANALYSIS *m*/*z* [ion]** |
|---|---|---|---|---|
| | **R2x** | **C6** | ***N9*** | |
| **III** | H | Cl | cyclopentyl | 223.1 [M+H]⁺ |
| **IV** | H | Cl | tetrahydrofuran-2-yl | 155.1 [M-THF+H]⁺ |
| **V** | H | Cl | tetrahydrofuran-3-yl | 225.1 [M+H]⁺ |
| **VI** | H | Cl | tetrahydro-2*H*-pyran-2-yl | 155.1 [M-THP+H]⁺ |
| **VII** | H | Cl | tetrahydro-2*H*-pyran-4-yl | 239.1 [M+H]⁺ |
| **VIII** | H | Cl | oxepan-2-yl | 253.4 [M+H⁺]- |
| **IX** | Cl | Cl | cyclopentyl | 256.8 [M+H]⁺ |
| **X** | Cl | Cl | tetrahydrofuran-2-yl | 188.8 [M-THF+H]⁺ |
| **XI** | Cl | Cl | tetrahydrofuran-3-yl | 259.4 [M+H]⁺ |
| **XII** | Cl | Cl | tetrahydro-2*H*-pyran-2-yl | 189.1 [M-THP+H]⁺ |
| **XIII** | Cl | Cl | tetrahydro-2*H*-pyran-4-yl | 273.4 [M+H]⁺ |
| **XIV** | Cl | Cl | oxepan-2-yl | 287.7 [M+H]⁺ |

**Table 2. Compounds 1-13 (C6 subst. = R¹-furfurylamino)**

| **Compound number** | **C2** | **PURINE SUBSTITUENT** | | **HMRS Calcd./Found [M+H]⁺** |
|---|---|---|---|---|
| | | **C6** | ***N9*** | |
| **1** | H | furfurylamino | cyclopentyl | 284.1506/284.1508 |
| **2** | H | furfurylamino | tetrahydrofuran-2-yl | 286.1299/286.1298 |
| **3** | H | furfurylamino | tetrahydrofuran-3-yl | 286.1299/286.1298 |
| **4** | H | furfurylamino | tetrahydro-2*H*-pyran-2-yl | 300.1455/300.1458 |
| **5** | H | furfurylamino | tetrahydro-2*H*-pyran-4-yl | 300.1455/300.1455 |
| **6** | Cl | furfurylamino | cyclopentyl | 318.1116/318.1116 |
| **7** | Cl | furfurylamino | tetrahydrofuran-2-yl | 320.0909/320.0910 |
| **8** | Cl | furfurylamino | tetrahydrofuran-3-yl | 320.0909/320.0909 |
| **9** | Cl | furfurylamino | tetrahydro-2*H*-pyran-2-yl | 334.1065/334.1072 |
| **10** | Cl | furfurylamino | tetrahydro-2*H*-pyran-4-yl | 334.1065/334.1067 |
| **11** | Cl | furfurylamino | oxepan-2-yl | 348.1222/348,1223 |
| **12** | Cl | 5-methylfurfurylamino | tetrahydrofuran-2-yl | 334.1065/334.1066 |
| **13** | Cl | 5-methylfurfurylamino | tetrahydro-2*H*-pyran-2-yl | 348.1222/348.1222 |

**Table 3. Compounds 14-61 (C6 subst. = R¹-furfurylamino).**

| **Compound number** | **PURINE SUBSTITUENT** | | | **HMRS Calcd/found [M+H]⁺** |
|---|---|---|---|---|
| | **C2** | **C6** | ***N9*** | |
| **14** | methylamino | furfurylamino | cyclopentyl | 313.1771/313.1769 |
| **15** | dimethylamino | furfurylamino | cyclopentyl | 327.1931/327.1931 |
| **16** | (2-aminoethyl)(methyl)amino | furfurylamino | cyclopentyl | 356.2193/356.2190 |
| **17** | 2-(dimethylamino)ethylamino | furfurylamino | cyclopenty | 370.2347/370.2347 |
| **18** | 2-aminoethylamino | furfurylamino | cyclopentyl | 342.2038/342.2038 |
| **19** | 2-hydroxyethylamino | furfurylamino | cyclopentyl | 343.1879/343.1879 |
| **20** | morpholino | furfurylamino | cyclopentyl | 369.2035/369.2035 |
| **21** | isopropoxy | furfurylamino | cyclopentyl | 342.1925/342.1927 |
| **22** | methylamino | furfurylamino | tetrahydrofuran-2-yl | 315.1564/315.1564 |
| **23** | dimethylamino | furfurylamino | tetrahydrofuran-2-yl | 329.1721/329.1720 |
| **24** | dimethylamino | 5-methylfurfurylamino | tetrahydrofuran-2-yl | 343.1877/343.1878 |
| **25** | methylamino | furfurylamino | tetrahydrofuran-3-yl | 315.1564/315.1565 |
| **26** | dimethylamino | furfurylamino | tetrahydrofuran-3-yl | 329.1721/329.1721 |
| **27** | (2-aminoethyl)(methyl)amino | furfurylamino | tetrahydrofuran-3-yl | 358.1986/358.1990 |
| **28** | 2-(dimethylamino)ethylamino | furfurylamino | tetrahydrofuran-3-yl | 372.2142/372.2143 |
| **29** | 2-aminoethylamino | furfurylamino | tetrahydrofuran-3-yl | 344.1832/344.1832 |
| **30** | 2-hydroxyethylamino | furfurylamino | tetrahydrofuran-3-yl | 345.1670/345.1671 |
| **31** | morpholino | furfurylamino | tetrahydrofuran-3-yl | 371.1826/371.1828 |
| **32** | isopropoxy | furfurylamino | tetrahydrofuran-3-yl | 344.1717/344.1707 |
| **33** | methylamino | furfurylamino | tetrahydro-2H-pyran-2-yl | 329.1721/329.1720 |
| **34** | dimethylamino | furfurylamino | tetrahydro-2H- | 343.1877/343.1886 |
| | | | pyran-2-yl | |
| **35** | propylamino | furfurylamino | tetrahydro-2*H-*pyran-2-yl | 357.2034/NA |
| **36** | dipropylamino | furfurylamino | tetrahydro-2*H-*pyran-2-yl | 399.2503/NA |
| **37** | benzylamino | furfurylamino | tetrahydro-2*H-*pyran-2-yl | 405.2034/NA |
| **38** | furfurylamino | furfurylamino | tetrahydro-2*H-*pyran-2-yl | 395.1826/NA |
| **39** | cyclopentylamino | furfurylamino | tetrahydro-2*H-*pyran-2-yl | 383.2190/383.2190 |
| **40** | pyrrolidin-1-yl | furfurylamino | tetrahydro-2*H-*pyran-2-yl | 369.2034/369.2034 |
| **41** | 2-aminoethylamino | furfurylamino | tetrahydro-2*H-*pyran-2-yl | 358.1986/358.1992 |
| **42** | 6-aminohexylamino | furfurylamino | tetrahydro-2*H-*pyran-2-yl | 414.2612/NA |
| **43** | (2-aminoethyl)(methyl)amino | furfurylamino | tetrahydro-2*H-*pyran-2-yl | 372.2146/372.2146 |
| **44** | 2-(dimethylamino)ethylamino | furfurylamino | tetrahydro-2*H-*pyran-2-yl | 386.2299/386.2308 |
| **45** | 2-hydroxyethylamino | furfurylamino | tetrahydro-2*H-*pyran-2-yl | 359.1826/359.1827 |
| **46** | 3-methoxypropylamino | furfurylamino | tetrahydro-2*H-*pyran-2-yl | 387.2139/NA |
| **47** | morpholino | furfurylamino | tetrahydro-2*H-*pyran-2-yl | 385.1983/385.1978 |
| **48** | thiomorpholino | furfurylamino | tetrahydro-2*H-*pyran-2-yl | 401.1754/401.1754 |
| **49** | isopropoxy | furfurylamino | tetrahydro-2*H-*pyran-2-yl | 358.1874/358.1870 |
| **50** | methylamino | 5-methylfurfurylamino | tetrahydro-2*H-*pyran-2-yl | 343.1877/343.1877 |
| **51** | dimethylamino | 5-methylfurfurylamino | tetrahydro-2*H-*pyran-2-yl | 357.2034/357.2036 |
| **52** | methylamino | furfurylamino | tetrahydro-2*H-*pyran-4-yl | 329.1721/329.1711 |
| **53** | dimethylamino | furfurylamino | tetrahydro-2*H-*pyran-4-yl | 343.1877/343.1879 |
| **54** | (2-aminoethyl)(methyl)amino | furfurylamino | tetrahydro-2*H-*pyran-4-yl | 372.2142/372.2145 |
| **55** | 2-(dimethylamino)ethylamino | furfurylamino | tetrahydro-2*H-*pyran-4-yl | 386.2299/386.2299 |
| **56** | 2-aminoethylamino | furfurylamino | tetrahydro-2*H-*pyran-4-yl | 372.2142/372.2145 |
| **57** | 2-hydroxyethylamino | furfurylamino | tetrahydro-2*H-*pyran-4-yl | 359.1826/359.1830 |
| **58** | morpholino | furfurylamino | tetrahydro-2*H-*pyran-4-yl | 385.1983/385.1986 |
| **59** | isopropoxy | furfurylamino | tetrahydro-2*H-*pyran-4-yl | 358.1874/358.1867 |
| **60** | methylamino | furfruylamino | oxepan-2-yl | 343.1877/343.1877 |
| **61** | dimethylamino | furfurylamino | oxepan-2-yl | 357.2034/357.2034 |

The compounds were synthesized from 6-chloropurine (**A**) or 2,6-dichloropurine (**B**) as starting building blocks, respectively. 2,3,4,5-Tetrahydrooxepine was prepared according to the previously published protocol (Liu et al., 2010 [doi: 10.1002/chem.200903441]).

Substituents to the *N*9 position of puring ring were introduced either by TFA catalysed hydroamination using 2,3-dihydrofuran, 3,4-dihydro-2*H*-pyran or 2,3,4,5-tetrahydrooxepine in dry EtOAc or by Mitsunobu alkylation using appropriate alcohol, PPh₃, and DIAD in anhydrous THF providing intermediates (**III-XIV**). Later, chlorine atom at C6 position of purine moiety was substituted by furfurylamine/5-methylfurfurylamine in presence of TEA as auxiliary base under conventional heating or in microwave-assisted reactions giving compounds (**1-13**). Chlorine atom in C2 position of compounds **IX-XIV** was substituted under various conditions such as: a) by heating *neat* or in appropriate solvent in the excess of corresponding amine (e.g. ethane-1,2-diamine, hexane-1,6-diamine, 2-aminoethanol, etc.); b) by heating with solution of corresponding amine (e.g. methylamine, dimethylamine); c) heating *neat* or in appropriate solvent in the presence of corresponding amine and auxiliary base such as DIPEA (e.g. morpholine, thiomorpholine, cyclopentylamine, *N,N-*dimethylethylamine, etc.), or d) by heating with corresponding alcoholate.

Functionalization of purine moiety of A or B at *N*9 position by hydroamination - general procedure:
TFA (1.5 equiv.) was dropwise added at 0 °C under argon atmosphere to a suspension of **A**/**B`** (1 equiv.), and 2,3-dihydrofuran/3,4-dihydro-2*H*-pyran/2,3,4,5-tetrahydrooxepine (1.3 equiv.) in dry EtOAc (**A**/**B** concentration, 0.5 M). Reaction mixture was allowed to warm up to room temperature and stirred for additional 1 h. Later, pH of the mixture was alkalized by 25% water ammonia/water (2:3, v/v) to pH 8-9. The layers were separated, and the water phase was re-extracted by EtOAc. Combined organic layers were washed with brine, dried over Na₂SO₄, filtered and concentrated under reduced pressure. Solid product was obtained after trituration with Et₂O/PE mixture.

### EXAMPLE 1: 2,6-dichloro-9-(tetrahydrofuran-2-yl)-9H-purine (IV)

White solid, chemical formula: C₉H₈Cl₂N₄O, yield: 98%. HPLC-UV/VIS retention time, purity (min, %): 21.08, 99.7. ESI⁺-MS *m*/*z* (intensity, ion): 188.8 (100, [³⁵Cl,³⁵Cl-M-THF+H]⁺), 190.8 (68, [³⁵Cl,³⁷Cl-M-THF+H]⁺), 192.8 (12, [³⁷Cl,³⁷Cl-M-THF+H]⁺). ¹H NMR (500 MHz, DMSO-*d₆*) δ (ppm): 2.00-2.08 (m, 1H, THF H4), 2.12-2.20 (m, 1H, THF H4'), 2.42-2.46 (m, 2H, THF H3, THF H3'), 3.93 (q, *J =* 7.5 Hz, 1H, THF H5), 4.18 (td, *J =* 7.9, 5.7 Hz, 1H, THF H5'), 6.33 (dd, *J =* 5.8, 4.3 Hz, 1H, THF H2), 8.82 (s, 1H, pur H8). ¹³C NMR (125 MHz, DMSO-*d*₆) δ (ppm): 23.9 (THF C4), 31.4 (THF C3), 69.3 (THF C5), 85.6 (THF C2), 131.1 (pur C5), 146.5 (pur C8), 149.7 (pur C6), 150.9 (pur C2), 152.6 (pur C4).

### EXAMPLE 2: 2,6-dichloro-9-(tetrahydro-2H-pyran-2-yl)-9H-purine (VI)

White solid, chemical formula: C₁₀H₁₀Cl₂N₄O, yield: 88%. HPLC-UV/VIS retention time, purity (min; %): 22.87, 98.4. ESI⁺-MS m/z (intensity, ion): 189.1 (100, [³⁵Cl,³⁵Cl-M-THP+H]⁺), 191.0 (73, [³⁵Cl,³⁷Cl-M-THP+H]⁺), 193.0 (16, [³⁷Cl,³⁷Cl-M-THP+H]⁺). ¹H-NMR (500 MHz, DMSO-*d*₆) δ (ppm): 1.56-1.61 (m, 2H, THP H5, THP H5'), 1.70-1.80 (m, 1H, THP H4), 1.94-2.01 (m, 2H, THP H3, THP H4'), 2.21-2.29 (m, 1H, THP H3'), 3.70-3.76 (m, 1H, THP H6), 4.01 (dd, *J =* 10.7, 1.5 Hz, 1H, THP H6'), 5.73 (dd, *J =* 10.9, 2.0 Hz, 1H, THP H2'), 8.95 (s, 1H, pur H8). ¹³C-NMR (125 MHz, DMSO-*d*₆) δ (ppm): 22.0 (THP C4), 24.3 (THP C5), 29.6 (THP C3), 67.7 (THP C6), 81.6 (THP C2), 130.5 (pur C5), 146.4 (pur C8), 149.9 (pur C6), 151.2 (pur C2), 152.8 (pur C4).

Functionalization of purine moiety of **A** or **B** at *N*9 position by Mitsunobu alkylation - general procedure:
DIAD (2 equiv.) was dropwise added under argon atmosphere and room temperature to a suspension of **A**/**B** (1 equiv.), R³-OH (2 equiv.), and triphenylphosphine (2 equiv.) in dry THF (**A**/**B** concentration, 0.15 M). Reaction mixture was stirred at room temperature for 1 h and then concentrated under reduced pressure. The majority of triphenylphosphine oxide was removed by crystallization from toluene. Filtrates were concentrated under reduced pressure and product was purified by silica gel column chromatography using either CHCl₃/MeOH, PE/EtOAc or Et₂O as a mobile phase or eventually obtained after recrystallization from MeOH.

### EXAMPLE 3: 2,6-dichloro-9-cyclopentyl-9H-purine (III)

Purified by crystallization from MeOH. White solid, chemical formula: C₁₀H₁₀Cl₂N₄, yield: 68%. HPLC-UV/VIS retention time, purity (min; %): 25.48, 98.2. ESI⁺-MS m/z (intensity, ion): 256.8 (100, [³⁵Cl,³⁵Cl-M+H]⁺), 258.8 (78, [³⁵Cl,³⁷Cl-M+H]⁺), 260.8 (18, [³⁷Cl,³⁷Cl-M+H]⁺). ¹H-NMR (500 MHz, DMSO-*d*₆) δ (ppm): 1.65-1.74 (m, 2H, cP H3), 1.83-1.91 (m, 2H, cP H3), 1.97-2.03 (m, 2H, cP H2), 2.15-2.22 (m, 2H, cP H2), 4.89-4.95 (m, 1H, cP H1), 8.82 (s, 1H, pur H8). ¹³C-NMR (125 MHz, DMSO-*d*₆) δ (ppm): 23.5 (2× C, cP C3), 31.8 (2× C, cP C2), 56.4 (cP C1), 130.8 (pur C5), 147.1 (pur C8), 149.5 (pur C6), 150.6 (pur C2), 153.3 (pur C4).

### EXAMPLE 4: 2,6-dichloro-9-(tetrahydro-2H-pyran-4-yl)-9H-purine (VIII)

White solid, chemical formula: C₁₀H₁₀Cl₂N₄O, yield: 63%. HPLC-UV/VIS retention time, purity (min; %): 19.95, 99.9. ESI⁺-MS m/z (intensity, ion): 273.4 (100, [³⁵Cl,³⁵Cl-M+H]⁺), 275.4 (68, [³⁵Cl,³⁷Cl-M+H]⁺), 277.4 (14, [³⁷Cl,³⁷Cl-M+H]⁺). ¹H-NMR (500 MHz, DMSO-*d*₆) δ (ppm): 1.98 (ddd, *J =* 12.3, 3.9, 1.6 Hz, 2H, THP H3), 2.18 (qd, *J =* 12.4, 4.6 Hz, 2H, THP H3'), 3.52 (td, *J =* 11.9, 1.8 Hz, 2H, THP H2), 3.99 (dd, *J =* 11.5, 4.4 Hz, 2H, THP H2'), 4.73 (tt, *J =* 12.1, 4.2 Hz, 1H, THP H4), 8.88 (s, 1H, pur H8). ¹³C-NMR (125 MHz, DMSO-*d*₆) δ (ppm): 31.9 (2× C, THP C3), 52.1 (THP C4), 66.0 (2× C, THP C2), 130.7 (pur C5), 146.9 (pur C8), 149.6 (pur C6), 150.6 (pur C2), 153.0 (pur C4).

Functionalization of purine intermediates (III-XIV) at C6 position - general procedures:
A mixture of purine intermediate **III-VIII** (1 eq.), furfurylamine/5-methylfurfurylamine (1.5 eq.), and TEA (2.5 eq.) in MeOH/*n*-PrOH (**III-VIII** concentration, 0.25 M) was heated at 120 °C in microwave reactor CEM Discovery SP for 15 min. The mixture was concentrated under reduced pressure. The residue was diluted with water and extracted by EtOAc. Combined organic extracts were washed with brine, dried over Na₂SO₄, filtered and concentrated under reduced pressure. Resulting residue was purified by silica gel column chromatography using CHCl₃/MeOH or PE/EtOAc as a mobile phase with gradient elution.

### EXAMPLE 5. N-(furan-2-ylmethyl)-9-(tetrahydrofuran-3-yl)-9H-purin-6-amine (compound 3)

Purified by column chromatography using CHCl₃/MeOH (100:1, v/v) with MeOH gradient. White solid, chemical formula: C₁₄H₁₅N₅O₂, yield: 65%. HPLC-UV/VIS retention time, purity (min, %): 14.99, 99.9. ESI⁺-MS m/z (intensity, ion): 286.2 (100, [M+H]⁺). HRMS (ESI⁺-TOF) m/z calcd. for C₁₄H₁₅N₅O₂ [M+H]⁺ 286.1299, found 286.1298. ¹H-NMR (500 MHz, DMSO-*d*₆) δ (ppm): 2.25-2.31 (m, 1H, THF H4), 2.43-2.48 (m, 1H, THF H4'), 3.85 (td, *J =* 8.6, 5.6 Hz, 1H, THF H5), 3.92-3.98 (m, 2H, THF H2, THF H2'), 4.09 (td, *J =* 8.1, 6.9 Hz, 1H, THF H5'), 4.67 (bs, 2H, CH₂-fur), 5.15-5.19 (m, 1H, THF H3), 6.21 (d, *J* = 2.8 Hz, 1H, fur H3), 6.34 (dd, *J =* 3.4, 1.8 Hz, 1H, fur H4), 7.52 (dd, *J =* 1.8, 0.9 Hz, 1H, fur H5), 8.16 (s, 1H, pur H8), 8.24 (bs, 2H, pur NH, pur H2). ¹³C-NMR (125 MHz, DMSO-*d*₆) δ (ppm): 31.8 (THF C4), 36.4 (CH₂-fur), 54.0 (THF C3), 66.5 (THF C5), 71.7 (THF C2), 106.6 (fur C3), 110.4 (fur C4), 119.3 (pur C5), 138.9 (pur C8), 141.7 (fur C5), 148.7 (pur C4), 152.1 (pur C2), 153.0 (fur C2), 154.1 (pur C6).

Functionalization of purine intermediates (**III-XIV**) at C6 position - general procedures:
A mixture of purine intermediate **III-XIV** (1 eq.), furfurylamine/5-methylfurfurylamine (1.2 eq.), and TEA (2.5 eq.) in *n*-PrOH (**III-XIV** concentration, 0.25 M) was heated at 100 °C in a reaction flask with reflux condenser or sealed tube for 4 h. The mixture was concentrated under reduced pressure. The residue was diluted with water and extracted by EtOAc. Combined organic extracts were washed with brine, dried over Na₂SO₄, filtered and concentrated under reduced pressure. Resulting residue was purified by silica gel column chromatography using CHCl₃/MeOH or PE/EtOAc as a mobile phase with gradient elution.

### EXAMPLE 6. 2-chloro-N-[(5-methylfuran-2-yl)methyl]-9-(tetrahydro-2H-pyran-2-yl)-9H-purin-6-amine (compound 13)

Purified by column chromatography using CHCl₃/MeOH (100:1, v/v) with MeOH gradient. White solid, chemical formula: C₁₆H₁₈ClN₅O₂, yield: 96%. HPLC-UV/VIS retention time, purity (min, %): 26.98, 99.9. ESI⁺-MS m/z (intensity, ion): 264.7 (91, [³⁵Cl-M-THP+H]⁺), 266.7 (47, [³⁷Cl-M-THP+H]⁺), 348.8 (100, [³⁵Cl-M+H]⁺), 350.8 (64, [³⁷Cl-M+H]⁺). HRMS (ESI⁺-TOF) m/z calcd. for C₁₆H₁₈ClN₅O₂ [M+H]⁺ 348.1222, found 348.1222. ¹H-NMR (500 MHz, DMSO-*d*₆) δ (ppm): 1.53-1.61 (m, 2H, THP H5, THP H5'), 1.67-1.76 (m, 1H, THP H4), 1.91-1.95 (m, 2H, THP H3, THP H4'), 2.17-2.27 (m, 4H, THP H3', fur-CH₃), 3.65-3.70 (m, 1H, THP H6), 3.98 (dd, *J =* 10.9, 1.7 Hz, 1H, THP H6), 4.55 (bd, *J =* 5.5 Hz, 2H, CH₂-fur), 5.56 (dd, *J =* 11.0, 1.5 Hz, 1H, THP H2), 5.96 (bs, 1H, fur H4), 6.11 (d, *J =* 3.1 Hz, 1H, fur H3), 8.38 (s, 1H, pur H8), 8.73 (t, *J =* 5.5 Hz, 1H, pur NH). ¹³C-NMR (125 MHz, DMSO-*d*₆) δ (ppm): 13.2 (fur-CH₃), 22.3 (THP C4), 24.4 (THP C5), 29.9 (THP C3), 36.7 (CH₂-fur), 67.6 (THP C6), 80.8 (THP C2), 106.4 (fur C4), 107.9 (fur C3), 118.1 (pur C5), 139.5 (pur C8), 149.4 (pur C4), 150.1 (fur C2), 150.5 (fur C5), 153.1 (pur C2), 154.6 (pur C6).

Functionalization of 2-chloro-9-substituted derivatives of *N*⁶-furfuryladenine at C2 position - general procedure 1:
A suspension of 2-chloro-9-substituted derivatives of *N*⁶-furfuryladenine (1 eq.) and corresponding amine (20-30 eq.) under nitrogen atmosphere was heated in a pressure tube at 165 °C for 3 h. The mixture was diluted with water and extracted by EtOAc. Combined organic extracts were washed with brine, dried over Na₂SO₄, filtered and concentrated under reduced pressure. Resulting residue was purified by silica gel column chromatography using CHCl₃/MeOH or PE/EtOAc as a mobile phase with gradient elution.

### EXAMPLE 7: 2-({6-[(furan-2-ylmethyl)amino]-9-(tetrahydro-2H-pyran-2-yl)-9H-purin-2-yl}amino)ethan-1-ol (compound 45)

Purified by column chromatography using CHCl₃/MeOH (100:1, v/v) with MeOH gradient. White solid, chemical formula: C₁₇H₂₂N₆O₃, yield: 42%. HPLC-UV/VIS retention time, purity (min, %): 21.22, 99.4. ESI⁺-MS m/z (intensity, ion): 358.9 (100, [M+H]⁺). HRMS (ESI⁺-TOF) m/z calcd. for C₁₇H₂₂N₆O₃ [M+H]⁺ 359.1826, found 359.1827. ¹H-NMR (500 MHz, DMSO-*d*₆) δ (ppm): 1.52-1.59 (m, 2H, THP H5, THP, H5'), 1.62-1.72 (m, 1H, THP H4), 1.86 (dd, *J =* 12.7, 2.3 Hz, 1H, THP H3), 1.93 (ddd, *J =* 12.9, 5.3, 3.1 Hz, 1H, THP H4'), 2.17 (qd, *J =* 12.1, 3.7 Hz, 1H, THP H3'), 3.34 (q, *J =* 5.5 Hz, 2H, -NHCH₂CH₂OH), 3.51 (t, *J* = 6.1 Hz, 2H, -NHCH₂CH₂OH), 3.57-3.62 (m, 1H, THP C6), 3.97 (dd, *J =* 11.0, 1.8 Hz, 1H, THP H6'), 4.62 (bs, 3H, CH₂-fur, -NHCH₂CH₂OH), 5.41 (dd, *J =* 11.0, 1.8 Hz, 1H, THP H2), 6.23 (d, *J =* 2.8 Hz, 1H, fur H3), 6.28 (bs, 1H, -NHCH₂CH₂OH), 6.35 (dd, *J =* 3.1, 1.8 Hz, 1H, fur H4), 7.51 (dd, *J =* 1.7, 0.8 Hz, 1H, fur H5), 7.71 (bs, 1H, pur NH), 7.88 (s, 1H, pur H8). ¹³C-NMR (125 MHz, DMSO-*d*₆) δ (ppm): 22.6 (THP C4), 24.5 (THP C5), 30.1 (THP C6), 36.3 (CH₂-fur), 43.9 (-NHCH₂CH₂OH), 60.3 (-NHCH₂CH₂OH), 67.5 (THP C6), 80.3 (THP C2), 106.5 (fur C3), 110.3 (fur C4), 113.1 (pur C5), 135.1 (pur C8), 141.6 (fur C5), 150.6 (pur C4), 153.3 (fur C2), 154.2 (pur C6), 159.1 (pur C2).

Functionalization of 2-chloro-9-substituted derivatives of *N*⁶-furfuryladenine at C2 position - general procedure 2:
A suspension of 2-chloro-9-substituted derivative of *N*⁶-furfuryladenine (1 eq.) and ethanolic solution of corresponding amine (20 eq., e.g. 5.6 M dimethylamine in abs. EtOH) was under nitrogen atmosphere heated in a pressure tube at 100 °C overnight. The mixture was concentrated under reduced pressure. Resulting residue was purified by silica gel column chromatography using CHCl₃/MeOH or PE/EtOAc as a mobile phase with gradient elution.

### EXAMPLE 8: 9-cyclopentyl-N⁶-(furan-2-ylmethyl)-N²,N²-dimethyl-9H-purine-2,6-diamine (compound 15)

Purified by column chromatography using CHCl₃/MeOH (100:1, v/v) with MeOH gradient. White solid, chemical formula: C₁₇H₂₂N₆O, yield: 83%. HPLC-UV/VIS retention time, purity (min, %): 26.14, 99.9. ESI⁺-MS *m*/*z* (intensity, ion): 327.3 (100, [M+H]⁺). HRMS (ESI⁺-TOF) *m*/*z* calcd. for C₁₇H₂₂N₆O [M+H]⁺ 327.1931, found 327.1931. ¹H-NMR (500 MHz, DMSO-*d*₆) δ (ppm): 1.61-1.69 (m, 2H, cP H3), 1.81-1.89 (m, 2H, cP H3'), 1.91-1.98 (m, 2H, cP H2), 2.04-2.10 (m, 2H, cP H2'), 3.06 (s, 6H, -N(CH₃)₂), 4.58 (bs, 2H, CH₂-fur), 4.67 (pent, *J =* 7.6 Hz, 1H, cP H1), 6.21 (d, *J =* 3.1 Hz, 1H, fur H3), 6.34 (dd, *J* = 3.1*,* 1.8 Hz, 1H, fur H4), 7.51 (d, *J =* 0.9 Hz, 1H, fur H5), 7.73 (bs, 1H, pur NH), 7.78 (s, 1H, pur H8). ¹³C-NMR (125 MHz, DMSO-*d₆*) δ (ppm): 23.8 (2× C, cP C3), 31.7 (2× C, cP C2), 36.4 (CH₂-fur), 36.9 (2× C, - N(CH₃)₂), 54.8 (cP C1), 106.5 (fur C3), 110.4 (fur C4), 113.1 (pur C5), 136.2 (pur C8), 141.6 (fur C5), 151.2 (pur C4), 153.6 (fur C2), 153.7 (pur C6), 158.8 (pur C2).

Functionalization of 2-chloro-9-substituted derivatives of *N*⁶-furfuryladenine at C2 position - general procedure 3:
A suspension of 2-chloro-9-substituted derivative of *N*⁶-furfuryladenine (1 eq.), corresponding amine (10 eq.), and DIPEA (5 eq.) was heated under nitrogen atmosphere in a pressure tube at 165 °C for 3 h. After cooling the mixture was diluted with water and extracted by EtOAc. Combined organic extracts were washed with brine, dried over Na₂SO₄, filtered and concentrated under reduced pressure. Resulting residue was purified by silica gel column chromatography using CHCl₃/MeOH or PE/EtOAc as a mobile phase with gradient elution. Reaction can be also performed in suitable solvents such as DMSO, *N,N*-dimethylformamid (DMF), *N*-methylpyrrolidone (NMP), or ethyleneglycol.

### EXAMPLE 9. N²-(2-(dimethylamino)ethyl)-N⁶-(furan-2-ylmethyl)-9-(tetrahydro-2H-pyran-4-yl)-9H-purine-2,6-diamine (compound 55)

Purified by preparative HPLC. White solid, chemical formula: C₁₉H₂₇N₇O₂, yield: 50%. HPLC-UV/VIS retention time, purity (min; %): 10.64, 99.9. ESI⁺-MS *m*/*z* (intensity, ion): 386.4 (100, [M+H]⁺). HRMS (ESI⁺-TOF) *m*/*z* calcd. for C₁₉H₂₇N₇O₂ [M+H]⁺ 386.2299, found 386.2299. ¹H-NMR (500 MHz, DMSO-*d*₆) δ (ppm): 1.87 (dd, *J =* 12.2, 2.1 Hz, 2H, THP H3), 2.14 (s, 8H, THP H3', -NHCH₂CH₂N(CH₃)₂), 2.37 (t, *J =* 6.9 Hz, 2H, -NHCH₂CH₂N(CH₃)₂), 3.32 (q, *J =* 6.4 Hz, 2H, -NHCH₂CH₂N(CH₃)₂), 3.43 (td, *J =* 11.8, 1.4 Hz, 2H, THP H2), 3.96 (dd, *J =* 11.3, 4.0 Hz, 2H, THP H2'), 4.38 (tt, *J =* 11.9, 4.1 Hz, 1H, THP H4), 4.59 (bs, 2H, CH₂-fur), 6.18 (bs, 1H, -NHCH₂CH₂N(CH₃)₂), 6.20 (dd, *J =* 3.2, 0.8 Hz, 1H, fur H3), 6.34 (dd, *J =* 3.4, 1.8 Hz, 1H, fur H4), 7.51 (dd, *J =* 1.8, 0.9 Hz, 1H, fur H5), 7.71 (bs, 1H, pur NH), 7.80 (s, 1H, pur H8). ¹³C-NMR (125 MHz, DMSO-*d*₆) δ (ppm): 32.1 (2× C, THP C3), 36.3 (CH₂-fur), 39.1 (-NHCH₂CH₂N(CH₃)₂), 45.3 (2× C, -NHCH₂CH₂N(CH₃)₂), 50.5 (THP C3), 58.4 (-NHCH₂CH₂N(CH₃)₂), 66.4 (2× C, THP C2), 106.4 (fur C3), 110.4 (fur C4), 113.7 (pur C5), 135.5 (pur C8), 141.6 (fur C5), 150.9 (pur C4), 153.5 (fur C2), 154.3 (pur C6), 158.9 (pur C2).

Functionalization of 2-chloro-9-substituted derivatives of *N*⁶-furfuryladenine at C2 position - general procedure 4:
A 60% dispersion of sodium hydride in mineral oil (3 eq.) was added carefully with cooling to the appropriate alcohol (2-chloro-9-substituted derivative of *N*⁶-furfurylamine concentration, 0.25 M). The mixture was stirred approximately for 30 min (until the end of gas development). 2-Chloro-9-substituted derivative of *N*⁶-furfuryladenine (1 eq.) was added and the mixture was stirred under nitrogen atmosphere at 95 °C overnight. After cooling, the mixture was concentrated under reduced pressure. The residue was treated with water and extracted by EtOAc. Combined organic extracts were washed with brine, dried over Na₂SO₄, filtered and concentrated under reduced pressure. Resulting residue was purified by silica gel column chromatography using CHCl₃/MeOH or PE/EtOAc as a mobile phase with gradient elution.

### Example 10. N-(furan-2-ylmethyl)-2-isopropoxy-9-(tetrahydro-2H-pyran-2-yl)-9H-purin-6-amine (compound 49)

Purified by column chromatography using CHCl₃/MeOH (100:1, v/v) with MeOH gradient. White solid, chemical formula: C₁₈H₂₃N₅O₃, yield: 56%. HPLC-UV/VIS retention time, purity (min, %): 23.50, 99.9. ESI⁺-MS *m*/*z* (intensity, ion): 358.4 (100, [M+H]⁺). HRMS (ESI⁺-TOF) *m*/*z* calcd. for C₁₈H₂₃N₅O₃ [M+H]⁺ 358.1874, found 358.1870. ¹H-NMR (500 MHz, DMSO-*d*₆) δ (ppm): 1.25 (dd, *J =* 6.1, 2.4 Hz, 6H, -OCH(CH₃)₂), 1.52-1.57 (m, 2H, THP H5, THP H5'), 1.64-1.73 (m, 1H, THP H4), 1.85-1.90 (m, 1H, THP H3), 1.94 (ddd, *J =* 12.9, 5.1, 3.6 Hz, 1H, THP H4'), 2.21 (qd, *J =* 12.1, 3.3 Hz, 1H, THP H3'), 3.60-3.65 (m, 1H, THP H6), 3.98 (dd, *J =* 10.7, 1.8 Hz, 1H, THP H6'), 4.60 (bs, 2H, CH₂-fur), 5.15 (sept, *J =* 6.1 Hz, 1H, -OCH(CH₃)₂), 5.47 (dd, *J* = 11.0, 1.8 Hz, 1H, THP H2), 6.20 (d, *J =* 3.1 Hz, 1H, fur H3), 6.35 (dd, *J =* 3.1, 1.8 Hz, 1H, fur H4), 7.53 (dd, *J =* 1.6, 0.8 Hz, 1H, fur H5), 8.12 (s, 1H, pur H8), 8.27 (bs, 1H, pur NH). ¹³C-NMR (125 MHz, DMSO-*d*₆) δ (ppm): 21.9 (2× C, - OCH(CH₃)₂), 22.5 (THP C4), 24.6 (THP C5), 30.0 (THP C3), 36.5 (CH₂-fur), 67.6 (THP C6), 68.6 (-OCH(CH₃)₂), 80.7 (THP C2), 106.5 (fur C3), 110.4 (fur C4), 115.1 (pur C5), 137.5 (pur C8), 141.8 (fur C5), 150.5 (pur C4), 152.9 (fur C2), 154.8 (pur C6), 160.6 (pur C2).

### EXAMPLE 11: Pharmaceutical preparations

### A1: Hard capsules

5000 capsules, each containing 0.25 g of the compound of formula I, are prepared as follows: Composition: compound of formula I: 1250 g; talc: 180 g; wheat starch: 120 g; magnesium stearate: 80 g; lactose 20 g.

Preparation procedure: Powdered and combined constituents are pushed through a sieve with a mesh size of 0.6 mm. A dose of 0.33 g of the mixture is transferred into a gelatin capsule using a capsule filling machine.

### A2: Hard capsules with N-(furan-2-ylmethyl)-9-(tetrahydro-2H-pyran-2-yl)-9H-purin-6-amine

5000 capsules, each containing 500 mg of *N*-(furan-2-ylmethyl)-9-(tetrahydro-2*H*-pyran-2-yl)-9*H*-purin-6-amine as active ingredient, are prepared by the following procedure: Composition: *N*-(furan-2-ylmethyl)-9-(tetrahydro-2*H*-pyran-2-yl)-9*H*-purin-6-amine: 2500 g; talc: 35 g; magnesium stearate: 10 g.

Preparation procedure: Homogenized components are pushed through a sieve with a mesh size of 0.6 mm. A dose of 0.51 g of the mixture is transferred into a gelatin capsule.

### A3: Soft capsules

5000 soft gelatin capsules, each containing 0.05 g of the compound of formula I as active ingredient, are prepared as follows:
Composition: compound of formula I: 250 g; lauroglycol: 21 g.
Preparation procedure: The powdered active ingredient is suspended in Lauroglycol^{®} and ground in a wet pulverizer to a particle size of about 1 to 3 mm. A 0.419 g dose of the mixture is then transferred into soft gelatin capsules using a capsule filling machine.

If the dose of the active ingredient is increased to 0.5 g, the amount of lauryl glycol is reduced accordingly.

### EXAMPLE 12: Compounds of formula I increase the period of thecircadian rhythm of human cells

Human osteosarcoma U2OS cells were grown in standard DMEM medium with 10% fetal bovine serum. U2OS cells were transduced with lentiviral particles containing a Bmal1-Luc fusion reporter and selected under Blasticidin. The cell line was clonally expanded and a single monoclonal cell line was used for further experiments. Cells were cultured in a 384-well plate in growth medium to 90+% confluence. Test compound or vehicle (DMSO) was applied to recording medium with 100 U/ml penicillin, 100 µg/ml streptomycin, 1x GlutaMAX, 10% fetal bovine serum, and 0.1 mM Luciferin-EF. Luminescence was recorded every hour for the next 144 hours. Circadian rhythm analysis was performed using cosinor analysis.

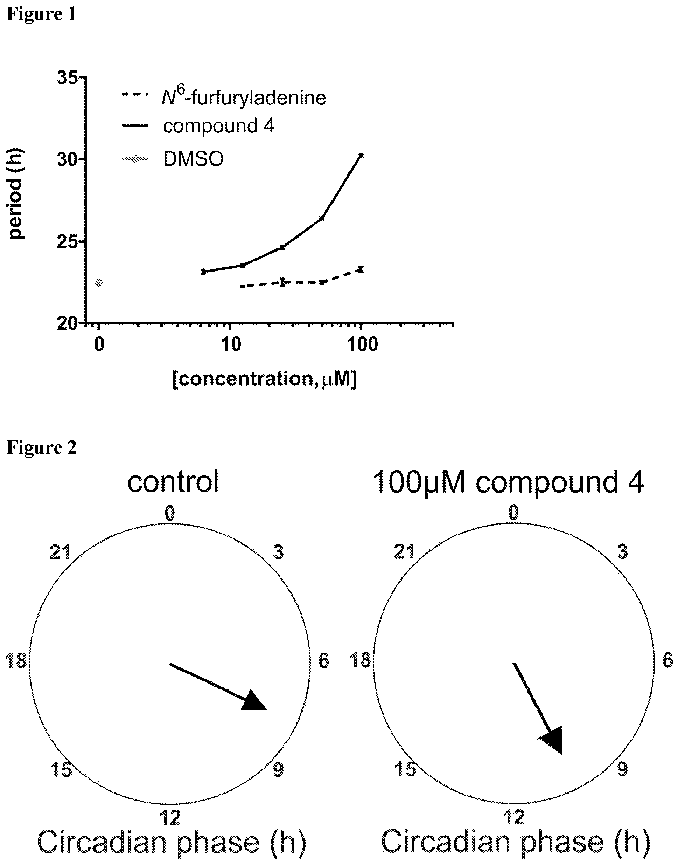

The length of the period was expressed relative to the period of cells affected only by DMSO vehicle (their period is therefore 1.00). The results are shown in Figure 2 and listed in Table 4. This experiment demonstrates that compounds of formula I increase the period of the circadian rhythm of human cells and can be used to manipulate the phase of the circadian rhythm, for example to synchronize the internal circadian clock with solar time. The overall effect on circadian oscillations unequivocally proves that the compounds of formula I can be also used to temporally modulate the concentration and activity of not only BMAL1, but also other components of the oscillator including the critical components PER2, CRY and CLOCK in time, as these are an essential part of the mechanism generating the rhythmic expression of the luminescent reporter used. The concentration and level of other proteins, whose rhythmic expression is controlled by an oscillator in the brain and peripheral tissues (so-called clock-controlled genes), are also necessarily affected. The influence of the compounds of formula I on the activity of oscillator components and subordinate genes also means influencing the physiological processes of the cell controlled by these genes and proteins. *N*⁶-furfuryladenine was used as a reference compound. An example shows that compounds of formula I show a significant effect on components of the circadian oscillator and the period of the circadian rhythm at concentrations at which *N*⁶-furfuryladenine has a minimal effect (Table 4). Compounds of formula I show a dose-dependent effect on the period length of the circadian rhythm and are more active than *N*⁶-furfuryladenine over a wide range of concentrations (Figure 1). The ability to shift the phase of the circadian cycle can be seen in Figure 2.

**Table 4. Effect of compounds of formula I on the length of the period of the circadian rhythm in the U2OS line. Control cells treated with DMSO vehicle have a relative period of 1.00.**

| Compound number or the name of the comparator compound | Period length ± standard error of the mean (expressed relative to control) at the indicated concentration |
|---|---|
| 1 | 10 µM: 1.19 ± 0.02 |
| 2 | 10 µM: 1.05 ± 0.01 |
| 3 | 10 µM: 1.06 ± 0.01 |
| 4 | 20 µM: 1.08 ± 0 |
| 5 | 10 µM: 1.05 ± 0.01 |
| 6 | 5 µM: 1.23 ± 0.03 |
| 7 | 10 µM: 1.13 ± 0.02 |
| 8 | 10 µM: 1.04 ± 0.01; 20 µM: 1.22 ± 0 |
| 9 | 10 µM: 1.09 ± 0.01; 20 µM: 1.11 ± 0.01 |
| 10 | 10 µM: 1.09 ± 0.01 |
| 12 | 10 µM: 1.16 ± 0.01 |
| 15 | 1 µM: 1.05 ± 0.01; 4 µM: 1.15 ± 0.01 |
| 16 | 10 µM: 1.26 ± 0.07; 20 µM: 1.95 ± 0.14 |
| 17 | 10 µM: 1.48 ± 0.28 |
| 18 | 1 µM: 1.16 ± 0.03 |
| 19 | 1 µM: 1.07 ± 0.01; 4 µM: 1.21 ± 0.01 |
| 20 | 1 µM: 1.09 ± 0.01; 4 µM: 1.67 ± 0.33 |
| 21 | 20 µM: 1.26 ± 0.06 |
| 22 | 10 µM: 1.06 ± 0 |
| 23 | 10 µM: 1.08 ± 0.01 |
| 24 | 10 µM: 1.03 ± 0; 20 µM: 1.09 ± 0.01 |
| 25 | 20 µM: 1.06 ± 0.01 |
| 27 | 20 µM: 1.25 ± 0.01 |
| 28 | 20 µM: 1.16 ± 0 |
| 29 | 20 µM: 1.09 ± 0 |
| 30 | 20 µM: 1.05 ± 0 |
| 31 | 20 µM: 1.13 ± 0.01 |
| 32 | 20 µM: 1.09 ± 0 |
| 33 | 5 µM: 1.04 ± 0 |
| 34 | 10 µM: 1.12 ± 0.01; 20 µM: 1.25 ± 0.03; 50 µM: 1.73 ± 0.6 |
| 35 | 10 µM: 1.05 ± 0.01 |
| 38 | 10 µM: 1.1 ± 0.02 |
| 39 | 20 µM: 1.05 ± 0.01 |
| 40 | 10 µM: 1.04 ± 0 |
| 41 | 1 µM: 1.03 ± 0.01; 5 µM: 1.07 ± 0; 20 µM: 1.32 ± 0.01 |
| 43 | 1 µM: 1.08 ± 0.01 |
| 44 | 1 µM: 1.05 ± 0.01; 5 µM: 1.28 ± 0.02 |
| 45 | 20 µM: 1.04 ± 0.01 |
| 46 | 20 µM: 1.05 ± 0.01 |
| 47 | 5 µM: 1.08 ± 0.01; 20 µM: 1.47 ± 0.03 |
| 49 | 20 µM: 1.14 ± 0.01 |
| 50 | 10 µM: 1.11 ± 0.01; 20 µM: 1.14 ± 0.02 |
| 52 | 20 µM: 1.08 ± 0.01 |
| 53 | 20 µM: 1.22 ± 0.01 |
| 54 | 5 µM: 1.14 ± 0.02 |
| 55 | 4 µM: 1.16 ± 0.01 |
| 56 | 20 µM: 1.26 ± 0.02 |
| 57 | 20 µM: 1.16 ± 0.01 |
| 58 | 20 µM: 1.42 ± 0.04 |
| 59 | 20 µM: 1.2 ± 0.01; 20 µM: 1.11 ± 0.02 |
| 60 | 5 µM: 1.04 ± 0; 20 µM: 1.15 ± 0.01 |
| 61 | 10 µM: 1.04 ± 0.01; 20 µM: 1.15 ± 0.02 |
| *N⁶*-furfuryladenine | 20 µM: 1.02 ± 0.01 |
| *N*⁶-(5-methylfurfuryl)adenine | 50 µM: 1.01 ± 0.02 |
| 2-chloro-*N*⁶-furfuryladenine | 20 µM: 1.00 ± 0.03 |
| 9-(tetrahydrofuran-2-yl)-9H-purin-6-amine | 20 µM: 0.98 ± 0.02 |
| 9-(ethoxymethyl)-*N*-(furan-2-ylmethyl)-9*H*-purin-6-amine | 50 µM: 1.01 ± 0.01 |
| 9-(2-ethoxyethyl)-*N*-(furan-2-ylmethyl)-9*H*-purin-6-amine | 50 µM: 0.99 ± 0.01 |
| *N*-(furan-2-ylmethyl)-9-(2-isopropoxyethyl)-9*H-*purin-6-amine | 50 µM: 1.01 ± 0.01 |
| 9-[2-(*tert*-butoxy)ethyl]-*N*-(furan-2-ylmethyl)-9*H-*purin-6-amine | 50 µM: 1 ± 0.01 |
| *N*-(furan-2-ylmethyl)-9-(3-methoxypropyl)-9*H*-purin-6-amine | 50 µM: 0.99 ± 0.01 |
| *N*-(furan-2-ylmethyl)-9-(4-methoxybutyl)-9*H*-purin-6-amine | 50 µM: 1 ± 0.01 |
| 9-benzyl-*N*-(furan-2-ylmethyl)-9*H*-purin-6-amine | 10 µM: 1 ± 0.02 |
| *N*-(furan-2-ylmethyl)-9-(methoxymethyl)-9*H*-purin-6-amine | 50 µM: 1.01 ± 0.01 |

### EXAMPLE 13: Compounds of formula I increase the period of the circadian rhythm of fibroblasts

NIH3T3 mouse embryonic fibroblasts were grown in standard DMEM with 10% fetal bovine serum. NIH3T3 cells were transfected with 1 µg of Per2-Luc reporter using GeneJuice and selected under hygromycin. The cell line was clonally expanded and a single monoclonal cell line was used for further experiments. Cells were cultured in a 96-well plate in growth medium to 90+% confluence. Test compound or vehicle (DMSO) was applied to recording medium with 100 U/ml penicillin, 100 µg/ml streptomycin, 1x GlutaMAX, 10% fetal calf serum and 0.1 mM Luciferin-EF (Promega Madison, WI, USA). Luminescence was recorded every hour for the next 144 hours. Circadian rhythm analysis was performed using cosinor analysis. Period length was expressed relative to the period of cells affected only by DMSO vehicle (their period length is therefore 1.00). The results are shown in Table 5.

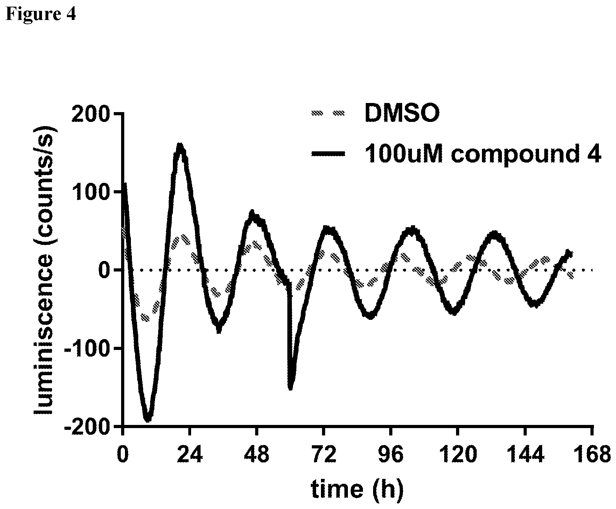

The experiment demonstrates that compounds of formula I extend the period of the circadian rhythm of mouse cells and can be used to manipulate the phase of the circadian rhythm, for example to synchronize the internal circadian clock with solar time. The overall effect on circadian oscillations unequivocally proves that these compounds can also be used for temporal modulation of the concentration and activity not only of PER2, but also of other components of the oscillator, including the critical components BMAL1, CRY and CLOCK, because these are a necessary part of the mechanism that generates rhythmic expression of the luminescent reporter used. The concentration and level of other proteins, whose rhythmic expression is controlled by an oscillator in the brain and peripheral tissues (so-called clock-controlled genes), are also necessarily affected. The effect of substitution compounds of formula I on the activity of oscillator components and subordinate genes also means influencing the physiological processes of the cell controlled by these genes and proteins. *N*⁶-furfuryladenine was used as a reference substance. Table 5 shows that the compounds of formula I show a significant effect on the components of the circadian oscillator and the period of the circadian rhythm at concentrations in which *N*⁶-furfuryladenine has a much smaller effect. Figure 4 shows that the effect of the compounds of formula I on the period of the circadian rhythm is dose-dependent and significantly greater than that of *N*⁶-furfuryladenine over a wide range of concentrations.

**Table 5. Effect of compounds of formula I on the length of the period of the circadian rhythm in the NIH3T3 line. Control cells treated with DMSO vehicle have a relative period of 1.00.**

| Compound number or the name of the comparator compound | Period length ± standard error of the mean (expressed relative to the control) at the indicated concentration |
|---|---|
| 1 | 10 µM: 1.17 ± 0.01; 50 µM: 1.54 ± 0.01 |
| 4 | 50 µM:1.23 ± 0.03 |
| 6 | 10 µM: 1.27 ± 0.01 |
| 10 | 10 µM: 1.14 ± 0.01 |
| 13 | 10 µM: 1.19 ± 0.01 |
| 15 | 0.83 µM: 1.08 ± 0.05; 50 µM: 1.56 ± 0.07 |
| 16 | 0.83 µM: 1.05 ± 0.01; 10 µM: 2.02 ± 0.06 |
| 17 | 10 µM: 1.71 ± 0.04; 50 µM: 2.17 ± 0.1 |
| 18 | 10 µM: 1.19 ± 0; 50 µM: 2.19 ± 0.35 |
| 20 | 0.83 µM: 1.04 ± 0.01; 50 µM: 1.55 ± 0.03 |
| 22 | 50 µM: 1.22 ± 0.02 |
| 23 | 10 µM: 1.07 ± 0.01; 50 µM: 1.36 ± 0.04 |
| 24 | 50 µM: 1.24 ± 0.07 |
| 33 | 50 µM: 1.18 ± 0.02 |
| 34 | 50 µM: 2.3 ± 0.03 |
| 35 | 50 µM: 1.38 ± 0.09 |
| 38 | 50 µM: 1.22 ± 0.06 |
| 44 | 50 µM: 1.72 ± 0.04 |
| 45 | 50 µM: 1.32 ± 0.01 |
| 46 | 50 µM: 1.27 ± 0.04 |
| 50 | 50 µM: 1.6 ± 0.02 |
| 51 | 0.83 µM: 1.05 ± 0.02; 10 µM: 1.08 ± 0.02; 50 µM: 1.74 ± 0.2 |
| 60 | 50 µM: 1.42 ± 0.04 |
| *N⁶*-furfuryladenine | 50 µM: 1.06 ± 0.02 |
| 9-(ethoxymethyl)-*N*-(furan-2-ylmethyl)-9*H*-purin-6-amine | 50 µM: 1 ± 0.03 |
| 9-(2-ethoxyethyl)-*N*-(furan-2-ylmethyl)-9*H*-purin-6-amine | 50 µM: 0.97 ± 0.03 |
| *N*-(furan-2-ylmethyl)-9-(2-isopropoxyethyl)-9*H*-purin-6-amine | 50 µM: 0.95 ± 0.02 |
| 9-[2-(*tert*-butoxy)ethyl]-*N*-(furan-2-ylmethyl)-9*H*-purin-6-amine | 50 µM: 0.9 ± 0.06 |
| *N*-(furan-2-ylmethyl)-9-(3-methoxypropyl)-9*H*-purin-6-amine | 50 µM: 0.93 ± 0 |
| *N*-(furan-2-ylmethyl)-9-(4-methoxybutyl)-9*H*-purin-6-amine | 50 µM: 0.97 ± 0.03 |
| 9-benzyl-*N*-(furan-2-ylmethyl)-9*H*-purin-6-amine | 10 µM: 1 ± 0.02 |
| *N*-(furan-2-ylmethyl)-9-(methoxymethyl)-9*H*-purin-6-amine | 50 µM: 0.96 ± 0.02 |

### EXAMPLE 14: Effect of compounds of formula I on increasing the period and modulation of the phase of the circadian rhythm of suprachiasmatic nucleus, the central circadian oscillator

The compounds of formula I increase the period and modulate the phase of circadian expression of the PER2::Luc clock reporter in the explanted suprachiasmatic nuclei (SCN) of the hypothalamus, the central clock of mammals, of transgenic mouse line.

Male and female mPer2Luc mice (strain B6.129S6-Per2tm1Jt / J, JAX, USA) (Yoo et al., 2004; doi: 10.1073/pnas.0308709101) were maintained in a light / dark cycle with 12 hours of light and 12 hours of darkness (LD12: 12), killed between 12:00 and 15:00, i.e. 6-9 hours after turning on the lights, by rapid cervical dislocation under isoflurane anesthesia, their brains were removed and 250 µm thick SCN slices in ice-cold HBSS medium were prepared using a vibratome (Leica, Wetzlar, Germany). Two explants containing SCN from each brain were prepared. Individual SCN explants were then placed on Millicell Culture Inserts (Merck, Darmstadt, Germany) inside 35 mm Petri dishes with test compound or DMSO vehicle in 1 ml air-buffered recording medium supplemented with 100 U / ml penicillin, 100 ug / ml streptomycin (Sigma-Aldrich, St. Luis, MO, USA), 1x GlutaMAX (ThermoFisher, Waltham, MA, USA), 5% fetal bovine serum (Sigma) and 0.1 mM Luciferin-EF (Promega Madison, WI, USA). The dishes were sealed with vacuum Vaseline and glass coverslips and placed inside a LumiCycle (Actimetrics) for bioluminescence recording. Rhythm analysis was performed in Lumicycle Analysis software (Actimetrics).

The results are shown in Figures 4 and 5. The example demonstrates that compounds of formula I increase the circadian rhythm period in the complex tissue of the central pacemaker and can be used to manipulate the phase of the circadian rhythm, for example to synchronize the internal circadian clock with solar time. The overall effect on the circadian oscillator indirectly, but unequivocally, demonstrates that the compound can also be used to modulate the concentration and activity of other components of the oscillator, including the critical components Bmal, Per2, Cry and Clock over time. The concentration and level of other proteins, whose rhythmic expression is controlled by an oscillator in the brain and peripheral tissues (so-called clock-controlled genes), is also necessarily affected. The effect of compounds of formula I on the activity of the oscillator components and subordinate genes also entails influencing the physiological processes of the cell controlled by these genes and proteins.

### EXAMPLE 15: Effect of compound 4 on increasing the period and modulating the phase of the circadian rhythm of choroid plexus, a periferal circadian oscillator

Male mPer2Luc were kept and sacrificed under the same conditions as above. Their brains were removed, choroid plexus was dissected from the 4th ventricle in ice-cold HBSS medium and placed on Millicell Culture Inserts inside 6-well plate with test compound or DMSO vehicle in 1 ml air-buffered recording medium supplemented with 100 U / ml penicillin, 100 ug / ml streptomycin (Sigma-Aldrich, St. Luis, MO, USA), 1x GlutaMAX (ThermoFisher, Waltham, MA, USA), 1x B27 (ThermoFisher) and 0.1 mM Luciferin-EF (Promega Madison, WI, USA). The were placed inside Luminoview LV200 Miscroscope (Olympus, USA) with liquid-cooled EM-CCD camera (Hamamatsu) and luminescence was recorded by 10 min exposure every 1h.

The results are shown in Fig 6. They demonstrate that compound 4 increases the period and modulates the phase of circadian expression of the PER2::Luc clock reporter in the explanted choroid plexus (ChP) of 4th brain ventricle. ChP is an example of important circadian peripheral tissue and is responsible for rhythmic production of cerebrospinal fluid. ChP is the crucial part of the recently discovered glymphatic system in charge of brain waste clearance of metabolites and soluble proteins, including amyloid-beta.

The compound 4 increases the period and modulates the phase of circadian expression of the PER2::Luc clock reporter in the explanted choroid plexus (ChP) of 4th brain ventricle. ChP is an example of important circadian peripheral tissue and is responsible for rhythmic production of cerebrospinal fluid. ChP is the crucial part of the recently discovered glymphatic system in charge of brain waste clearance of metabolites and soluble proteins, including amyloid-beta.

The example demonstrates that compound 4 increases the circadian rhythm period in the complex peripheral tissue of choroid plexus and can be used to manipulate the phase of the circadian rhythm in this tissue, for example to synchronize the internal circadian clock with solar time. responsible for rhythmic production of cerebrospinal fluid. ChP is the crucial part of the recently discovered glymphatic system in charge of brain waste clearance of metabolites and soluble proteins, including amyloid-beta. Due to the highly rhythmic physiology of the ChP, it would be possible to manipulate the rhythmic production of cerebrospinal fluid in vivo by application of the effective compound.

## Claims

1. Heterocyclic compound of general formula Ia 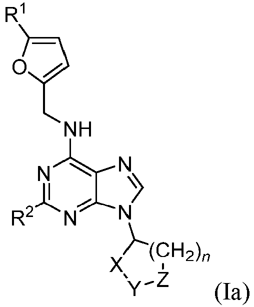 and their pharmaceutically acceptable salts,
wherein
- *n* is 1, X is CH₂ and each of Y, Z is selected from CH₂ and O, wherein exactly one of Y and Z is O; or
- *n* is 2, X is CH₂ and each of Y, Z is selected from CH₂ and O, wherein at most one of Y and Z is O; or
- *n* is 3, X is CH₂ and each of Y, Z is selected from CH₂ and O, wherein at most one of Y and Z is O;
and
R¹ is H or C1-C4 alkyl;
R² is selected from H, Cl, C1-C4 alkoxy and NR²¹R²²;
wherein R²¹ and R²² are independently selected from H, C3-C6 cycloalkyl and C1-C8 alkyl, wherein one carbon atom in the C1-C8 alkyl may optionally be replaced by one nitrogen atom or one oxygen atom;
or wherein R²¹ is H or C1-C4 alkyl and R²² is selected from C6-C10 aryl-methyl, C6-10 aryl-ethyl, C3-C6 heteroaryl-methyl, C3-C6 heteroaryl-ethyl, wherein the heteroaryl group contains 1-2 heteroatoms selected from O, S, N;
or wherein R²¹ and R²² together with the nitrogen atom to which they are bound form a 3- to 8-membered ring optionally containing one further heteroatom selected from O, S, N.

2. Heterocyclic compound of general formula I 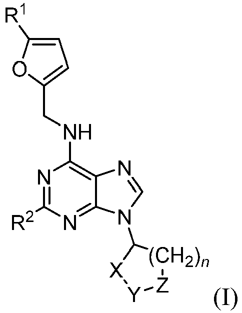 and their pharmaceutically acceptable salts,
wherein
each of X, Y, Z is selected from CH₂ and O, wherein at most one of X, Y, Z is O;
*n* is 1 or 2 or 3;
R¹ is H or C1-C4 alkyl;
R² is selected from H, Cl, C1-C4 alkoxy and NR²¹R²²,
wherein R²¹ and R²² are independently selected from H, C3-C6 cycloalkyl and C1-C8 alkyl, wherein one carbon atom in the C1-C8 alkyl may optionally be replaced by one nitrogen atom or one oxygen atom;
or wherein R²¹ is H or C1-C4 alkyl and R²² is selected from C6-C10 aryl-methyl, C6-10 aryl-ethyl, C3-C6 heteroaryl-methyl, C3-C6 heteroaryl-ethyl, wherein the heteroaryl group contains 1-2 heteroatoms selected from O, S, N;
or wherein R²¹ and R²² together with the nitrogen atom to which they are bound form a 3- to 8-membered ring optionally containing one further heteroatom selected from O, S, N;
for use in the prevention and/or therapy of circadian rhythm diseases.

3. Heterocyclic compound of formula I for use according to claim 2, wherein the use is therapeutic, and the disease to be treated is selected from the group of hereditary advanced sleep phase syndrome - FASPS, advanced sleep phase disorder - ASPD, irregular sleep phase syndrome and free running sleep phase.

4. Heterocyclic compound of formula I for use according to claim 2, wherein the use is therapeutic, and the disease to be treated is abnormal circadian activity associated with neurodegeneration selected from the group consisting of mild cognitive impairment, Alzheimer's disease, and Smith-Magenis syndrome, and wherein the compound of formula I is administered to subjects exhibiting abnormal circadian activity as a result of a shortened period of the circadian cycle and/or advanced phase of the circadian rhythm.

5. Heterocyclic compound of formula I for use according to claim 2, wherein the use is therapeutic or prophylactic, and the disease to be treated is selected from the group consisting of jet lag disorder, social jet lag, and shift-work disorder.

6. Heterocyclic compound of formula I as defined in claim 2 for use in synchronizing a patient's optimal circadian time and solar time for the application of therapy, in particular chemotherapy, radiotherapy or surgery.

7. A pharmaceutical composition, **characterized in that** it contains at least one compound of formula Ia according to claim 1 or a pharmaceutically acceptable salt or solvate thereof, and at least one pharmaceutically acceptable carrier.

## Patentansprüche

1. Heterocyclische Verbindung der allgemeinen Formel Ia 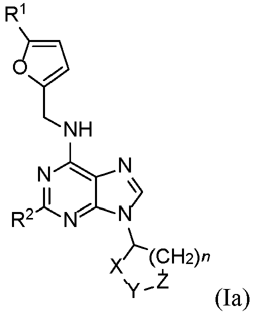 und deren pharmazeutisch annehmbare Salze,
wobei
- *n* = 1, X = CH₂ und Y und Z jeweils CH₂ oder O sind, wobei genau eines von Y und Z ein O-Atom ist; oder
- *n* = 2, X = CH₂ und Y und Z jeweils CH₂ oder O sind, wobei höchstens eines von Y und Z ein O-Atom ist; oder
- *n* = 3, X = CH₂ und Y und Z jeweils CH₂ oder O sind, wobei höchstens eines von Y und Z ein O-Atom ist;
und
R¹ H oder C1-C4-Alkyl ist;
R² ausgewählt ist aus H, Cl, C1-C4-Alkoxy und NR²¹R²²;
wobei R²¹ und R²² unabhängig voneinander ausgewählt sind aus H, C3-C6-Cycloalkyl und C1-C8-Alkyl, wobei in der C1-C8-Alkylgruppe ein Kohlenstoffatom optional durch ein Stickstoffatom oder Sauerstoffatom ersetzt sein kann;
oder wobei R²¹ H oder C1-C4-Alkyl ist und R²² ausgewählt ist aus C6-C10-Arylmethyl, C6-C10-Arylethyl, C3-C6-Heteroarylmethyl und C3-C6-Heteroarylethyl, wobei die Heteroarylgruppe 1-2 Heteroatome ausgewälht aus O, S, N enthält;
oder wobei R²¹ und R²² zusammen mit dem Stickstoffatom, an dem sie gebunden sind, einen 3- bis 8-gliedrigen Ring bilden, der optional ein weiteres Heteroatom ausgewählt aus O, S, N enthält.

2. Heterocyclische Verbindung der allgemeinen Formel I 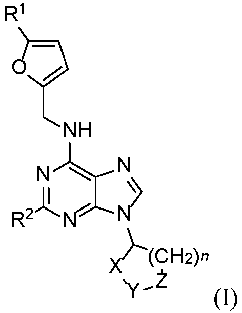 und deren pharmazeutisch annehmbare Salze,
wobei
jedes von X, Y und Z ausgewählt ist aus CH₂ und O, wobei höchstens eines von X, Y und Z ein O-Atom ist;
*n* 1 oder 2 oder 3 ist;
R¹ H oder C1-C4-Alkyl ist;
R² ausgewählt ist aus H, Cl, C1-C4-Alkoxy und NR²¹R²²;
wobei R²¹ und R²² unabhängig voneinander ausgewählt sind aus H, C3-C6-Cycloalkyl und C1-C8-Alkyl, wobei in der C1-C8-Alkylgruppe ein Kohlenstoffatom optional durch ein Stickstoffatom oder Sauerstoffatom ersetzt sein kann;
oder wobei R²¹ H oder C1-C4-Alkyl ist und R²² ausgewählt ist aus C6-C10-Arylmethyl, C6-C10-Arylethyl, C3-C6-Heteroarylmethyl und C3-C6-Heteroarylethyl, wobei die Heteroarylgruppe 1-2 Heteroatome ausgewählt aus O, S, N enthält;
oder wobei R²¹ und R²² zusammen mit dem Stickstoffatom, an dem sie gebunden sind, einen 3- bis 8-gliedrigen Ring bilden, der optional ein weiteres Heteroatom ausgewählt aus O, S, N enthält;
zur Anwendung in der Prävention und/oder Therapie von Störungen des zirkadianen Rhythmus.

3. Heterocyclische Verbindung der Formel I zur Anwendung gemäß Anspruch 2, wobei die Anwendung therapeutisch ist und die zu behandelnde Erkrankung aus der Gruppe der erblichen, fortschreitenden Schlafphasenstörung (FASPS), der fortschreitenden Schlafphasenstörung (ASPD), des irregulären Schlafphasen-Syndroms und der freien Schlafphasen-Störung ausgewählt ist.

4. Heterocyclische Verbindung der Formel I zur Anwendung gemäß Anspruch 2, wobei die Anwendung therapeutisch ist und die zu behandelnde Erkrankung eine mit Neurodegeneration verbundene Störung des zirkadianen Rhythmus ist, ausgewählt aus der Gruppe der leichten kognitiven Beeinträchtigung, der Alzheimer-Krankheit und dem Smith-Magenis-Syndrom, und wobei die Verbindung der Formel I an Patienten verabreicht wird, die aufgrund einer verkürzten Periode des zirkadianen Rhythmus und/oder einer vorzeitigen Phase des zirkadianen Rhythmus eine gestörte zirkadiane Aktivität aufweisen.

5. Heterocyclische Verbindung der Formel I zur Anwendung gemäß Anspruch 2, wobei die Anwendung therapeutisch oder prophylaktisch ist und die zu behandelnde Erkrankung aus der Gruppe der Jetlag-Störung, des sozialen Jetlags und der Schichtarbeitsstörung ausgewählt ist.

6. Heterocyclische Verbindung der Formel I gemäß Anspruch 2 zur Synchronisierung der optimalen zirkadianen Zeit eines Patienten mit der Sonnenzeit für die Anwendung einer Therapie, insbesondere Chemotherapie, Strahlentherapie oder Operation.

7. Pharmazeutische Zusammensetzung, **gekennzeichnet dadurch dass** sie mindestens einer Verbindung der Formel Ia gemäß Anspruch 1 oder einem pharmazeutisch akzeptablen Salz oder Solvat davon sowie mindestens einem pharmazeutisch akzeptablen Träger enthält.

## Revendications

1. Composé hétérocyclique de formule générale Ia 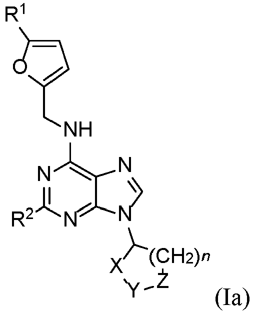 et leurs sels pharmaceutiquement acceptables,
où
- *n* est égal à 1, X est CH₂ et Y et Z sont chacun CH₂ ou O, avec la condition qu' exactment un des Y ou Z soit O; ou
- *n* est égal à 2, X est CH₂ et Y et Z sont chacun CH₂ ou O, avec la condition que, au maximum, l'un des Y ou Z soit O; ou
- *n* est égal à 3, X est CH₂ et Y et Z sont chacun CH₂ ou O, avec la condition que, au maximum, l'un des Y ou Z soit O;
et
R¹ est H ou un groupe alkyle en C1-C4;
R² est H, Cl, un groupe alcoxy en C1-C4 ou NR²¹R²²;
où R²¹ et R²² sont indépendamment choisis du H, un groupe cycloalkyle en C3-C6 et un groupe alkyle en C1-C8, où le groupe alkyle en C1-C8 peut éventuellement contenir un atome d'azote ou d'oxygène à la place d'un atome de carbone;
ou bien R²¹ est H ou un groupe alkyle en C1-C4 et R²² est choisi d'un groupe arylméthyle en C6-C10, aryléthyle en C6-C10, hétéroarylméthyle en C3-C6 et hétéroaryléthyle en C3-C6, le groupe hétéroaryle contenant 1 à 2 hétéroatomes choisis parmi O, S et N;
ou bien R²¹ et R²², avec l'atome d'azote auquel ils sont liés, forment un cycle à 3 à 8 chaînons, pouvant éventuellement contenir un hétéroatome supplémentaire choisi parmi O, S et N.

2. Composé hétérocyclique de formule générale I 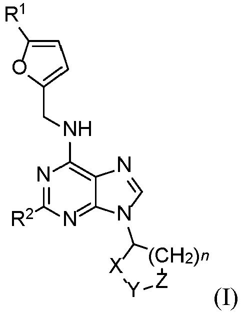 et leurs sels pharmaceutiquement acceptables,
où
chacun des X, Y et Z est choisi parmi CH₂ et O, sachant que au maximum l'un des X, Y ou Z peut être O;
n peut prendre les valeurs 1, 2 ou 3 ;
R¹ est H ou un groupe alkyle en C1-C4;
R² est H, Cl, un groupe alcoxy en C1-C4 ou NR²¹R²²;
où R²¹ et R²² sont indépendamment choisis du H, un groupe cycloalkyle en C3-C6 et un groupe alkyle en C1-C8, où le groupe alkyle en C1-C8 peut éventuellement contenir un atome d'azote ou d'oxygène à la place d'un atome de carbone;
ou bien R²¹ est H ou un groupe alkyle en C1-C4 et R²² est choisi d'un groupe arylméthyle en C6-C10, aryléthyle en C6-C10, hétéroarylméthyle en C3-C6 et hétéroaryléthyle en C3-C6, le groupe hétéroaryle contenant 1 à 2 hétéroatomes choisis parmi O, S et N;
ou bien R²¹ et R²², avec l'atome d'azote auquel ils sont liés, forment un cycle à 3 à 8 chaînons, pouvant éventuellement contenir un hétéroatome supplémentaire choisi parmi O, S et N; pour utilisation dans la prévention et/ou le traitement des troubles du rythme circadien.

3. Composé hétérocyclique de formule I pour utilisation selon la revendication 2, destiné à un usage thérapeutique, la maladie à traiter étant choisie parmi le syndrome familial de phase avancée du sommeil (FASPS), le trouble de phase avancée du sommeil (ASPD), le syndrome de phase irrégulière du sommeil et le cycle de sommeil à phase libre.

4. Composé hétérocyclique de formule I pour utilisation selon la revendication 2, destiné à un usage thérapeutique, la maladie à traiter étant une anomalie de l'activité circadienne associée à une neurodégénérescence, choisie parmi les troubles suivants déficience cognitive légère, maladie d'Alzheimer et syndrome de Smith-Magenis, où le composé de formule I est administré à des sujets présentant une anomalie de l'activité circadienne due à une période raccourcie du cycle circadien et/ou à une phase avancée du rythme circadien.

5. Composé hétérocyclique de formule I pour utilisation selon la revendication 2, destiné à un usage thérapeutique ou prophylactique, la maladie à traiter étant choisie parmi le décalage horaire, le décalage horaire social et les troubles liés au travail posté.

6. Composé hétérocyclique de formule I pour utilisation selon la revendication 2, où l'utilisation est pour synchroniser le rythme circadien optimal du patient avec l'heure solaire, en vue d'une thérapie, notamment une chimiothérapie, une radiothérapie ou une intervention chirurgicale.

7. Composition pharmaceutique **caractérisée par le fait qu'**elle contient au moins un composé de formule Ia selon la revendication 1, ou un sel ou un solvate pharmaceutiquement acceptable de ce composé, ainsi qu'au moins un excipient pharmaceutiquement acceptable.
